# EUROPEAN PATENT SPECIFICATION

(11) **EP 0 456 332 B1**
(45) Date of publication and mention of the grant of the patent: **09.08.2000**
(21) Application number: 91201769.6
(22) Date of filing: 13.03.1985
(51) Int. Cl.: C12N 15/25, C12P 21/02, C07K 14/545, C12N 1/00

(54) **Purified interleukin 1 and DNA coding for interleukin 1 and their preparation, vectors containing such DNA and their preparation and use in transforming hosts to permit expression of interleukin 1**
Gereinigtes Interleukin-1 und für Interleukin-1 kodierende DNS und deren Herstellung, solche DNS enthaltende Vektoren und deren Herstellung und Verwendung zum Transformieren von Wirten, die die Expression von Interleukin-1 zulassen
Interleukine-1 purifiée et ADN codant pour l'interleukine-1 et leur préparation, vecteurs contenant cet ADN et leur préparation et utilisation dans la transformation des hôtes permettant l'expression d'interleukine-1

(30) Priority: 19.06.1984 US 622201; 27.07.1984 US 635006; 26.11.1984 US 674555; 30.11.1984 US 676533; 31.12.1984 US 687646
(43) Date of publication of application: 13.11.1991
(62) Divisional of application: 85301752.3
(73) Proprietor: Cistron Biotechnology Inc., Pine Brook, NJ 07058 (US)
(72) Inventor: Cerretti, Douglas P., Seattle, Washington 989133 (US); Conlon III, Paul J., Walnut Creek, CA 94596 (US); Cosman, David J., Bainbridge Island, Washington 98110 (US); Grabstein, Kenneth H., Mercer Island, Washington 90040 (US); Hopp, Thomas P., Seattle, Washington 98116 (US); Kronheim, Shirley R., Seattle, Washington 98125 (US); Larsen, Alf D., Seattle, Washington 98102 (US); March, Carl J., Bainbridge Island, Washington 98110 (US); Price, Virgina L., Seattle, Washington 98102 (US)
(74) Representative: Perry, Robert Edward

(56) References cited:
- EP-A- 0 161 901
- EP-A- 0 187 991
- LYMPHOKINE RESEARCH, vol. 3, no. 4, summer 1984, page 285, Mary Ann Liebert, Inc. Publishers, New York, US; P.E. AURON et al.: "Molecular cloning of human interleukin-1 (IL-1) cDNA"
- NATURE, vol. 302, 24th March 1983, pages 305-310, Macmillan Journals Ltd; T. TANIGUCHI et al.: "Structure and expression of a cloned cDNA for human interleukin-2"
- THE JOURNAL OF IMMUNOLOGY, vol. 122, no. 5, May 1979, pages 2112-2118, The Williams & Wilkins Co., US; A. TOGAWA et al.: "Characterization of lymphocyte-activating factor (LAF) produced by human mononuclear cells: Biochemical relationships of high and low molecular weight forms of LAF"
- J EXP MED 160 (Sept. 1984) 772-787

## Description

The present invention relates to the cloning of a gene for interleukin 1 (hereinafter "IL-1") by use of a synthetic oligonucleotide probe, and to the characterization of the screened IL-1 gene.

IL-1, formerly known in the literature as "lymphocyte activating factor" or "LAF," is a hormone produced by macrophages while undergoing an immune response. This protein factor regulates a wide range of immunological and non-immunological responses. For instance, it is considered that IL-1 mediates activities referred to as endogenous or leukocytic pyrogen, B-cell activating factor (BAF), epidermal cell thymocyte activating factor (ETAF), leukocyte endogenous mediator (LEM), bone resorption factor active in rheumatoid arthritis, and a variety of other activities. As such, IL-1 shows promise for therapeutic mediation of immune response, which is defined herein to include the aforementioned activities.

Although researchers have identified many of the biological properties of IL-1, the chemical nature of this hormone is not well understood. To date, this has been hampered, at least in part, by the unavailability of sufficient quantities of IL-1 in purified form to carry out necessary investigations.

Attempts have been made in the past to purify and partially characterize IL-1 derived from both human and murine sources. For instance, Mizel, 122 J. Immunol. 2167-2172 (1979), reported the production of murine IL-1 from the macrophage cell line, P388D₁. The IL-1 from the culture fluid was subjected to ammonium sulfate precipitation, diethyl amino ethyl ("DEAE") cellulose column chromatography, ultrafiltration and Sephacryl S200 column chromatography. The resulting active fractions were found to have a molecular weight in the range of 12,000 to 16,000 daltons. Through isoelectrofocusing ("IEF") in polyacrylamide gels, the pI of the IL-1 was found to be in the range of from 5.0 to 5.4.

In a subsequent communication Mizel et al., 126 J. Immunol. 834-837 (1981), discussed purifying IL-1 from the same P388D₁ cell line as used in Mizel, supra, to "apparent homogeneity" by ammonium sulfate precipitation, phenyl Sepharose chromatography, Ultrogel AcA54 gel filtration chromatography and preparative flat-bed IEF. The resulting IL-1 was found to have a pI of about 4.9 to 5.1, and a molecular weight of approximately 14,000 daltons.

Blyden et al., 118 J. Immunol. 1631-1638 (1977), disclosed a protocol for concentrating IL-1 prepared from human peripheral blood leukocytes by Sephadex G-100 column chromatography. This procedure was reported to result in a four-to-five fold concentration of the crude IL-1. DEAE-Bio-Gel A anion exchange chromatography was employed to remove the albumin from the serum used during the preparation of the crude IL-1. Next, the collected active fractions were adsorbed onto a hydroxyapatite column and then applied to a CM-Bio-Gel A cationic exchange resin. The researchers reported that by these procedures, about 20% of the initial IL-1 was recovered. The resulting IL-1 was found to have a molecular weight of about 13,000 daltons and a pI of approximately 6.8 to 7.2.

Crude IL-1 prepared from human leukocytes by Togawa et al., 122 J. Immunol. 2112-2118 (1979) was initially processed by membrane filtration and then applied to a Bio-Gel P-100 chromatography column which disclosed two major peaks of activity, one in the range of from 12,000 to 22,000 daltons and another in the range of about 50,000 to 70,000 daltons. Active fractions in the lower molecular weight region were pooled and then applied to a Blue Sepharose column, a DEAE-cellulose ion-exchange chromatography column and to a hydroxylapatite chromatography column. Togawa et al. discovered that if the lower molecular weight IL-1 activity resulting from each of these procedures was reconstituted with 2% human serum, concentrated and rechromatographed on Bio-Gel P-100, a significant portion of the higher molecular weight activity appeared.

Lachman, 42 Federation Proceedings 2639-2645 (1983), reported preparing IL-1 from peripheral blood monocytes or leukemic cells obtained from acute monocytic leukemia or acute myelomonocytic leukemia patients. Hollow fiber diafiltration and ultrafiltration were used to separate a lower molecular weight activity from most of the serum proteins. This lower weight activity was subjected to IEF in an Ampholine and sucrose gradient. From this procedure, the IL-1 activity was found to have a pI of about 6.8 to 7.2 and a molecular weight of about 11,000 daltons. Lachman reported that the overall recovery of IL-1 activity from the above procedures was poor, in the range of about 4%.

The availability of adequate quantities of homogeneous human IL-1 could be valuable in investigations and possible treatment of autoimmune disorders such as arthritis and lupus erythmatosis. Also, human IL-1 in greater purity and larger quantities than heretofore available, could prove useful in achieving successful wound and burn healing.

One potential method of providing relatively large quantities of homogeneous human IL-1 is through recombinant DNA techniques. Recombinant DNA techniques have been developed for economically producing a desired protein once the gene coding for the protein has been isolated and identified. A discussion of such recombinant DNA techniques for protein production is set forth in the editorial and supporting papers in Vol. 196 of Science (April 1977). However, to take advantage of the recombinant DNA techniques discussed in this reference, the gene coding for human IL-1 must first be isolated.

EP-A-0 161 901 discloses DNA encoding human IL-lβ.

In accordance with the present invention, a gene coding for human IL-1 was isolated from a cDNA library with a synthetic oligonucleotide probe corresponding to a portion of the amino acid sequence of human IL-1 as determined above. Total human RNA was extracted from cells thought to produce relatively high levels of IL-1. Polyadenylated mRNA was isolated from the total RNA extract. A cDNA library was constructed by reversed transcription of size separated polyadenylated mRNA with reverse transcriptase. The DNA was rendered double-stranded with DNA polymerase I and inserted into an appropriate cloning vector. Resultant recombinant cloning vectors were used to transform an appropriate host.

Transformed hosts were identified and grouped into pools. Plasmid DNA prepared from these pools was hybridized with the oligonucleotide probe that had been radiolabeled. The pool(s) of clones that gave a positive signal to the probe was identified and then the putative pool subdivided and the hybridization screen repeated. By this procedure, a single transformant was eventually identified. Plasmid DNA was prepared from this transform ant and characterized by restriction endonuclease digestion. The IL-1 gene was sequenced to establish its nucleic acid and amino acid compositions. Also the IL-1 gene was cloned in an E. coli/yeast cell system to express mature IL-1, and then biological assays were conducted to confirm that the expressed protein product was IL-1.

The details of typical embodiments of the present invention will be described in connection with the accompanying drawings, in which:
FIGURE 1 illustrates a partial structural formula for blue dye-ligand;
FIGURE 2 illustrates a partial structural formula for red dye-ligand;
FIGURE 3 illustrates a partial restriction map of the IL-1 gene;
FIGURE 4, containing two sheets, illustrates the nucleotide sequence and the corresponding amino acid sequence of the IL-1 gene as contained in the nucleotide fragment in FIGURE 3, with the nucleotides being numbered from the beginning of the sequence shown in FIGURE 4 and the amino acids being numbered from the mature NH₂-terminus of the protein, i.e., the Ala residue, as marked with an arrow, to the termination codon ATT at residue number 153; and,
FIGURE 5 illustrates the strategy employed to clone the coding region of the IL-1 gene in a shuttle vector used to transform yeast hosts to express functional IL-1.

### Preparation of IL-1

Crude preparations of IL-1 are prepared from peripheral blood leukocytes. The leukocytes are separated from whole blood by well known techniques, such as by centrifugation over a volume of Ficoll/Hypaque solution. The leukocytes removed from the blood are cultured in vitro in a culture medium containing an appropriate stimulating agent to induce IL-1 secretion. After an optimum culture period, the supernatant is harvested by centrifugation and stored until used.

Rather than being obtained from leukocytes removed from whole blood, IL-1 can alternatively be prepared from monocytes derived from any monocyte rich source. Such monocyte sources include monocytic leukemic spleen cells, lymph cells and alevolar macrophages.

The medium used to culture the peripheral blood leukocytes may consist of commercially available media, such as Eagle's Minimum Essential Medium ("MEM") or Roswell Park Memorial Institute ("RPMI") medium. Additives, which may be individually or in combination added to the culture medium, include glutamine, HEPES buffer and various antibiotics, such as gentamycin, penicillin and streptomycin. In the past, serum also has been commonly used as an additive. However, applicants have discovered that in the procedures of the present invention, purification of IL-1 from the culture supernatant is facilitated if serum is not used in the culture. Although not employing serum has been found to result in a three-to-five fold reduction in the quantity of IL-1 produced in culture, the absence of serum also results in a 100-fold reduction in total protein produced, which lessens the complications involved in the purification of the IL-1.

Preferable stimulating agents used in conjunction with the present invention include Staphylococcus aureus or lipopolysaccharide ("LPS") extracted from Escherichia coli ("E. coli"). In addition, phorbol esters, such as phorbal myristrate 13-acetate, may be employed as a stimulating agent.

The process of culturing the leukocytes to induce secretion of IL-1 may be carried out in various environmental conditions. Preferably, however, the cultures are maintained in the temperature range of approximately 35-38°C in a humidified atmosphere of approximately 5-10% CO₂ in air. The quantity of IL-1 released by stimulation of peripheral blood leukocytes with an activating agent varies with time. Applicants have found that optimum levels of IL-1 expression are reached at approximately 24 - 72 hours after stimulation.

### Assays/Analysis

A thymocyte proliferation assay, an IL-1 conversion assay and a protein assay are employed in conjunction with the present invention to monitor the IL-1 activity level and the protein content of the samples during the purification, cloning and IL-1 expression procedures of the present invention. Also, sodium dodecyl sulfate-polyacrylamide gel electrophoresis ("SDS-PAGE") and two-dimensional gel electrophoresis are used to analyze the IL-1 activity during the purification process.

### Thymocyte Proliferation Assay

This assay involves ascertaining the capacity of a sample of IL-1 to induce proliferation of thymocytes derived from CD-1 mice. In this assay, approximately 1 x 10⁶ thymocytes obtained from 10 to 12 week old CD-1 mice (Charles River Breeding Laboratories, Wilmington, MA) are seeded in round bottom microplate wells (Corning Plastics, Corning, New York) in the presence of three-fold serial dilutions of IL-1 containing samples. The thymocytes are cultured in 150 microliters ("ul") of MEM containing 50 units/milliliter ("U/ml") penicillin, 50 micrograms/milliliter ("ug/ml") streptomycin, 2 millimolar ("mM") glutamine, 0.2 mM gentamycin, 10 mM HEPES buffer, (jointly referred to as "Supplemented MEM"), pH 7.4, together with 3% v/v human serum and 10⁻⁵ molar ("M") 2-mercaptoethanol. The samples are cultured for 72 hours at 37°C in an atmosphere of 5% CO₂ in air. Thereafter the cultures are pulsed for approximately 4 hours with 0.5 microcuries ("uCi") of tritiated thymodine ("³H-Tdr"), (New England Nuclear, Boston, Massachusetts, 2 Ci/mM specific activity), after which the cultures are harvested onto glass fiber filter strips, for instance with the aid of a multiple-automated sample harvester. ³H-Tdr incorporation is then measured by liquid scintillation counting. Details of this procedure are disclosed in Gillis et al., 120 J. Immunol. 2027 (1978).

By this thymocyte proliferation assay procedure, only the CD-1 thymocytes cultured in the presence of IL-1 incorporate ³H-Tdr in a dose dependent manner. CD-1 cells cultured in the absence of IL-1 incorporate only background levels of ³H-Tdr. IL-1 activity is calculated from the linear portion of the ³H-Tdr incorporation data in a manner similar to the procedure used by Gillis et al., supra, for determining interleukin-2 activity. Units of IL-1 activity are determined as the reciprocal dilution of a sample which generates 50% of maximal thymocyte ³H-Tdr incorporation as compared to a laboratory standard. For example, if a sample generates 50% of maximal thymocyte ³H-Tdr incorporation at a dilution of 1:15, then one unit ("U") of IL-1 is found in 1/15 of the 150 ul assay volume, or 10 ul is said to contain one U of activity. The total sample would, therefore, contain 100 U [1,000 (ul/ml) 10 ul (per U)] of IL-1 activity/ml. See Gillis et al., supra.

### IL-1 Conversion Assay

A second alternative assay for IL-1 activity may be employed which takes advantage of the fact that IL-1 was found by applicants to convert an interleukin 2 ("IL-2") nonproducer murine tumor cell line, LBRM-33-145, to an IL-2 producer. In this assay LBRM-33-1A5 cells, ATCC No. CRL-8079, are inactivated by addition of 50 ug/ml of mitomycin C and incubated for 1 hour at 37°C. 100 ul of the inactivated LBRM-33-1A5 cells (5 x 10⁵ cells/ml) are cultured in 96-well flat-bottomed plates in the presence of an equal volume of the mitogen, phytohemagglutinin ("PHA") (1% final concentration) together with serial dilutions of IL-1 containing fluid samples. After 6-24 hours, the existence of IL-2 activity, generated by IL-1 triggered, mitomycin C - inhibited LBRM-33-1A5 cells (and thus IL-1 activity), is directly ascertained by adding 50 ul of IL-2 dependent CTLL-2 cells (8 x 10⁴ cells/ml). The microwell cultures are then incubated for 20 additional hours followed by a 4 hour pulse with 0.5 uCi of ³H-Tdr (New England Nuclear, Boston, MA, 2 Ci/mM specific activity). Thereafter, the thymidine-pulsed cultures are harvested onto glass fiber filter strips with the aid of a multiple automated sample harvester (MASH II; Microbiological Associates, Bethesda, MD). ³H-Tdr incorporation is measured by liquid scintillation counting. Details of this procedure are set forth in Gillis et al. supra, and in U.S. Patent 4,411,992. In this assay, only the CTLL-2 cells cultured in the presence of IL-2 incorporate ³H-Tdr in a dose dependent manner. CTLL-2 cells cultured in the absence of IL-2 (and thus IL-1) incorporate only background levels of ³H-Tdr. This "conversion" assay has the advantage of being quicker (completion within 24 hours) and approximately 1000 to 10,000 times more sensitive than the above-discussed thymocyte proliferation assay. Nevertheless, both the "conversion" and "proliferation" assays may be employed in conjunction with the present invention.

### Protein Assay

The protein content of the purification samples is determined by Biorad protein assay which is commercially available from Biorad, Richmond, California. This assay employs bovine serum albumin as a standard. The principles and details of this assay are discussed in Bradford, 72 Anal. Biochem. 248 (1976).

### Gel Electrophoresis

The culture supernatant and chromatography column fractions are analyzed by SDS-PAGE to monitor the purification procedures of the present invention. This assay is conducted according to the gel stacking procedure of Laemmli, 227 Nature (London) 680 (1970). The assay employs 0.75 mm SDS slab gels using a 10-20% gradient of polyacrylamide gel. The gels are run at a constant 30 mA current. The resulting gel samples are silver stained, such as by the method described in Oakley et al., 105 Anal. Biochem. 361(1980).

The particular assay samples that contain a high salt concentration are initially dialyzed against 0.001% SDS in 0.1 mM NH₄HCO₃ and then dried under a vacuum. The dried residue is dissolved in a reducing buffer (2% SDS), 1% 2-mercaptoethanol prior to the SDS-PAGE process.

### Two-Dimensional Polyacrylamide Gel Electrophoresis

After completion of the purification procedures of the present invention, the IL-1 is analyzed by two-dimensional polacrylamide gel electrophoresis by the method described in Sammons et al., 2 Electrophoresis 135 (1981). In the procedure lyophilized IL-1 samples are resuspended in 20 ul of SDS solubilization buffer composed of 1% (w/v) cyclohexy-laminoethane, 2% (w/v) SDS, 2% 2-mercaptoethanol, 10% glycerol in water. The samples are heated to 100°C for 10 minutes. After two hours of prefocusing, samples (solubilized for 10 min. in SDS at 100°C) are applied to the first dimension gel and focused for 20 hours at a constant voltage of 600 volts. The first dimension focusing gels are scanned directly with a pH gradient gel scanner. Thereafter the gels are rinsed in equilibration buffer [9.3% (v/v) glycerol; 50% (v/v) Tris-SDS buffer (30g Tris, 2g. DSD per liter, adjusted to pH 6.8 with concentrated HC); 1% (w/v) SDS; 0.8% (v/v) 2-mercaptoethanol in water] for 2 minutes, placed on top of the second dimension gel and then covered with low temperature melting agarose. The second dimension electrophoresis (10-20% linear gradient of acrylamide) is conducted at a constant current of 40 mA/gel until the dye front reaches the bottom of the gel. After fixation in 50% (v/v) ethanol and 10% (v/v) glacial acetic acid, the gels are stained by color silver nitrate method of Sammons et al., supra.

### Purification of IL-1

The supernatant resulting from the blood leukocyte culture as prepared by the above procedure is purified by cation exchange chromatography, anion exchange chromatography, and affinity chromatography employing a dye-ligand coupled to a column matrix. All chromatography fractions are assayed for IL-1 activity and protein concentration. Where appropriate, pH and conductivity are measured. Following each chromatography step, samples are analyzed by SDS-PAGE. In addition, after completion of the affinity chromatography procedure, active fractions are analyzed by two-dimensional polyacrylamide gel electrophoresis as discussed above.

A suitable column for the cation exchange chromatograpy process is composed of sulfopropyl Sephadex C-25 (Pharmacia Fine Chemicals, Piscataway, New Jersey). Preferably, the column is equilibrated with buffer prior to application of the IL-1 sample and then washed with the same or different buffer after the IL-1 sample has been applied to the column to remove nonbound protein without elution of IL-1 activity. Elution of the IL-1 from the column is carried out with a buffered elutant of sufficient pH to disassociate the IL-1 from the column.

The pooled active fractions from the cation exchange chromatography procedure are further purified by anion exchange chromatography. Applicants have found that a suitable column material for this purpose is DEAE-Sephacel. The DEAE-Sephacel column is equilibrated with a buffer and the sample concentrate applied to the column. Elution is initially carried out with the starting buffer and then subsequently with a linear salt gradient in the same buffer. Fractions are collected and analyzed as discussed above.

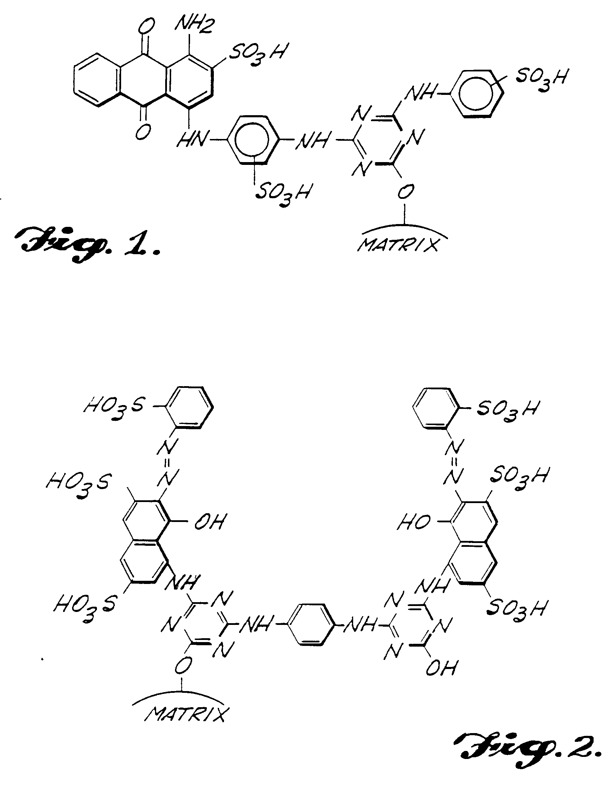

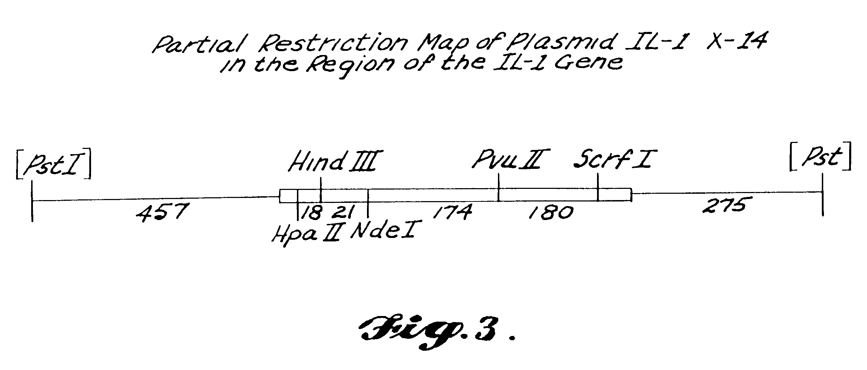

The IL-1 in the pooled active fractions from the DEAE-Sephacel column is further purified by affinity column chromatography employing a synthetic triazinyl textile dye-ligand coupled to a support matrix. Various dye colors may be employed including blue or red. The dye is coupled to an appropriate column matrix composed of, for instance, agarose, polyacrylamide, cellulose or silica-based inorganic materials via an ether linkage to the trazine ring or alternatively via a primary amine or an anthraquinone group of the dye. Rather than being bound directly to a support matrix, the dye can be bound to high molecular weight dextran with the dextran then immobilized to a column matrix. Partial chemical structures of blue and red dye-ligands coupled to a matrix are shown in FIGURES 1 and 2, respectively. It is to be understood that these dye structures can be modified to form analogs, for instance by exchanging the positions of the sulfonated anthraquinone group relative to the triazine ring or by substituting a sulfonate salt for the sulfonic acid substituents. See Fulton, Dye-Ligand Chromatography, Lexington, MA: Studio 6, Inc. (1980).

Prior to applying the IL-1 containing fractions purified over SP-Sephadex and DEAE-Sephacel to the dye-ligand column, it may be necessary to lower the ionic strength of the pooled active fractions. Also, the presence of a divalent cation, such as Mg⁺⁺ or Ca⁺⁺, may enhance the binding of IL-1 to the dye-ligand. The column is equilibrated with an appropriate buffer, such as Tris-HCL, and then the pooled DEAE fractions containing IL-1 activity are applied to the column. Thereafter, the column is washed with the same starting buffer and then elution is carried out with a linear salt gradient in the same buffer or a specific soluble ligand. Fractions are collected and analyzed as discussed above.

Applicants have discovered that red triazinyl textile dye, when used under the stated column conditions, is especially highly specific for binding IL-1. A commercial brand of this red dye corresponding to FIGURE 2 is "Procion" red (reactive red 120) (Imperial Chemical Industries). Applicants have also discovered that blue triazinyl textile dye when used under the stated column conditions is also highly specific for binding IL-1, i.e. approximately 80% as specific as red triazinyl textile dye. A commercial brand of blue dye is Cibacron® Blue 36A (Ciba AG).

By the aforementioned purification process, applicants have purified human IL-1 protein to greater than 99% purity while maintaining a high yield of about 53% from the starting supernatant. By the above-described assay procedures, applicants have determined that human IL-1 is composed of a singular molecular weight specie of approximately 17,500 daltons molecular weight. This molecular weight is substantially heavier than previously reported for either human or murine IL-1. Moreover, contrary to reports of other observers, no other molecular weight species of IL-1 were found by applicants. See Lachman, supra; Togawa et al., supra; Mizel et al., supra; and Blyden et al., supra. Nevertheless, because of the high yields of IL-1 experienced by applicants by use of the present invention, it is unlikely that any significant amounts of other lower molecular weight IL-1 species were lost during the aforementioned purification process. The true molecular weight for homogeneous human IL-1 is therefore 17,500 daltons.

### Amino Acid Composition Analysis

In addition to making possible the biological study of IL-1 free from contamination by other proteins, the ability to prepare homogeneous IL-1 has enabled applicants to determine the amino acid composition of the IL-1 molecule. This information may be employed to assist in the cloning of the IL-1 gene and the production of large quantities of pure IL-1 for clinical trials and ultimately for clinical use.

Samples of purified IL-1 from the affinity chromatography procedure are analyzed for amino acid composition with an automated analyzer using ninhydrin detection. Observed peaks are integrated with commercially available recording integrator. Through this technique, applicants have determined the amino acid composition of the IL-1 molecule as summarized in Table I in Example 4, below.

Since the amino acid residue cysteine (Cys) is unstable to hydrolysis, this residue is not detected by automated ninhydrin analysis. The presence of the Cys residue was detected by the amino acid sequencing analysis, discussed below. Also, automated ninhydrin analysis does not distinguish aspartic acid residues from asparagine residues nor does it distinguish glutamic acid residues from glutamine residues. However, from the amino acid sequencing analysis, discussed below, an asparagine residue and six glutamine residues were detected in a N-terminal portion of the IL-1 molecule (consisting of 42 amino acid residues). Thus, in Table I the aspartic acid and asparagine residues are listed together, as are the glutamic acid and glutamine residues.

### Amino Acid Sequence Analysis of N-Terminal Portion of IL-1 Molecule

Applicants have also investigated the amino acid sequence of the IL-1 molecule. Applicants have discovered that in the purified IL-1 prepared by the procedures set forth above, the N-terminus of this molecule is partially blocked. As such, the molecule is not readily amenable to the chemical analysis technique employed in automated amino acid sequencing apparatuses, and thus the amino acid sequence of the entire molecule could not be determined by standard analysis procedures. As a result, applicants employed a combination of two techniques to analyze the sequence of the N-terminal portion of the protein molecule.

As a first technique applicants subjected a rather large sample of over 11 ug of IL-1, as purified to homogeneity by the aforediscussed methods, to amino terminal Edman degradation sequence analysis with an automated sequencing apparatus. By this technique the first 20 residues of the N-terminal portion of the IL-1 molecule was found to be composed of the following sequence: NH₂-Ala-Pro-Val-Arg-Ser-Leu-Asn-Cys-Thr-Leu-Arg-Asp-Ser-Gln-Gln-Lys-Ser-Leu-Val-Met.

The 8th residue was deduced to be Cys. In the eighth cycle of the automated sequencing procedure no other residue was obtained in high yield, which points to the conclusion that the eighth residue is composed of Cys (which is not affirmatively detected by Edman degradation), a glycosylated threonine residue or a glycosylated serine (Ser) residue. These later two possibilities were elmininated since, as discussed below, no glucosamine or galactosamine was observed from the amino acid composition analysis. This leads to the conclusion that the 8th residue is composed of Cys.

As a second amino acid sequence analysis technique, applicants fractionated the molecule at the argine residues with the enzyme trypsin. To prevent the trypsin from also cleaving the IL-1 molecule at the lysine sites, the side chains of the lysine molecule were protected with a specific blocking agent. Preferably, the trypsin is treated with L-1-tosylamino-S-phenylethylchloromethylketone ("TPCK") to deactivate other contaminating enzymes, such as chymotryspin, that may also be present, thereby minimizing the possibility that the IL-1 protein will be cleaved at other residues. It is to be understood that rather than employing trypsin, other enzymes may be used to cleave the IL-1 molecules at other residue sites.

After cleavage of the IL-1 molecule, the resulting peptides were separated on the basis of hydrophobocity by HPLC procedures. The HPLC technique used in the present invention preferably employs a reversed phase, octadecyl bonded silica column having a pore size sufficiently large to be optimally utilized with the proteineaceous IL-1 peptides, i.e., a pore size of at least 300 A.

Suitable reversed phase HPLC columns for use in the practice of the present invention are articles of commerce. A preferred column for this purpose is the Vydac 218 TP reversed phase column commercially available from Separations Group, Hesperia, California. This column consists of octadecyl silane groups covalently bonded by means of a siloxane (silican-oxygen-silicaon) bond to the surface of the 300 A pore diameter silica gel which has been classified to a mean particle size of 5 microns. It is to be understood that the use of other reversed phase columns is within the scope of the present invention.

The IL-1 peptides that are bonded to the octadecyl column are eluted by the use of a linear gradient of acetonitrile. A preferred gradient for this purpose is a 0 to 95% (v/v) acetonitrile gradient in trifluoroacetic acid (TFA), pH 2.0.

The eluted peptides can be conveniently monitored with commercially available detection systems. For example, the relative protein concentration in the fractions eluted from the HPLC columns can be determined by measuring absorbance of the eluted material with an automated ultraviolet light spectrophotometer, at 230 nanometers wavelength. A suitable automated ultraviolet light absorbance detection apparatus is available from Waters Associates, Millford, Maine. Alternatively, protein elution can be monitored with an automated fluorescence detection system, as described by Stein and Moschera, 78 Meth. Enzymol. 435 (1981).

The eluted HPLC fractions are analyzed in sequence by gel eletrophoresis, discussed above, to determine the number of peptides contained in each of the HPLC fractions. Thereafter, the peptides are concentrated in vacuo and then analyzed for amino acid sequence. This is preferably carried out with an automated sequencing apparatus, which are articles of commerce. Through this technique, applicants have discovered that a major portion of the Il-1 molecule near the N-terminal portion of the human IL-1 molecule is composed of the following sequence of amino acid residues: -Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe.

The first four residues of the amino terminal portion of this amino acid fragment corresponds with the last four residues of the C-terminal portion of the sequence determined above by automated Edman degradation technique, thus leading to the conclusion that the first 42 residues of the N-terminal portion of the Il-1 molecule is composed of the following sequence: NH₂-Ala-Pro-Val-Arg-Ser-Leu-Asn-Cys-Thr-Leu-Arg-Asp-Ser-Gln-Gln-Lys-Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe

### Preparation of RNA from Human IL-1 Producing Cells

Total RNA from human IL-1-producing cells is extracted by standard methods, such as disclosed by Chirgwin et al., 18 Biochemistry 5294 (1979), and Maniatis et al., Molecular Cloning, a Laboratory Manual, Cold Spring Harbor Laboratory, Cold Spring Harbor, New York (1982).

As is well known, when extracting RNA from cells, it is important to minimize ribonuclease ("RNase") activity during the initial stages of extraction. One manner in which this is accomplished is to denature the cellular protein, including the RNase, at a rate that exceeds the rate of RNA hydrolysis by RNase. In the procedures of Chirgwin et al., supra, and Maniatis et al., supra at 196, this is carried out by use of guanidinium thiocyanate, together with a reducing agent, such as 2-mercaptoethanol (to break up the protein disulfide bonds). The RNA is isolated from the protein by standard techniques, such as phenol/chloroform extraction, ethanol precipitation or sedimentation through cesium chloride.

Next, polyadenylated mRNA is separated from the extracted protein. Although several techniques have been developed to carry out this separation process, one preferred method is to chromatograph the polyadenylated mRNA on oligo (dT)-cellulose as described by Edmonds et al., 68 Proc. Natl. Acad. Sci. 1336 (1971); Aviv and Leder, 69 Proc. Natl. Acad. Sci. 1408 (1972); and, Maniatis et al., supra at 197. The oligo (dT)-cellulose column is prepared with a loading buffer and then the mRNA applied to the column. Thereafter, the column is initially washed with a buffer solution to remove the unpolyadenylated mRNA and then the polyadenylated mRNA is eluted from the column with a buffered, low ionic strength eluent. The integrity of the polyadenylated mRNA is verified by gel electrophoresis.

The polyadenylated mRNA is then sized by electrophoresis through methylmercury agarose gel fractions corresponding to different size classes of mRNA and then translated in vitro, by use of standard rabbit reticulocyte lysates technique, such as described by: Palmiter, 248 J. Biol. Chem. 2095 (1973); Pelham and Jackson, 67 Eur. J. Biochem. 246 (1976); and, Lee et al., 253 J. Biol. Chem. 3494 (1978). Kits for the rabbit reticulocyte assay are commercially available from many sources, such as from Bethesda Research Laboratories, Gaithersburg, Maryland. Alternatively, the mRNA translation can be carried out by microinjection of the mRNA into frog Xenopus laevis ("X. laevis") oocytes using standard techniques, such as described by Stoma et al., 79 Meth. Enzym. 68 (1981). Fluids liberated by either reticulocyte lysate translations, or by mRNA microinjected oocytes are then tested for the presence of IL-1 activity by use of the assays discussed above. mRNA gel fractions which, when translated in vitro gave rise to IL-1 activity, are selected as a source of mRNA for cDNA construction.

In the X. laevis oocyte translation procedure, approximately 50 nanoliters ("nl") of mRNA (dissolved in sterile H₂O at a concentration of 0.5-1 mg/ml) is injected into each oocyte. The oocytes are harvested from X. laevis (Nasco, Fort Atkinson, Wisconsin) and incubated in 150 ml of oocyte incubation medium (88 mM NaCl, 1 mM KCl, 2.4 mM NaHCO₃, 0.82 mM MgS0₄ · 7 H₂O, 0.33 mM Ca (NO₃)₂ · 4H₂O, 0.41mM CaCl₂ · 6H₂O, 7.5 mM Tris base, 18 units/ml (11 ug/ml) penicillin G potassium, and 18 ug/ml streptomycin). The final pH of the medium is adjusted to 7.6 with HCl and then sterilized by filtration. After injection, the oocytes are placed in 0.1 ml of fresh oocyte incubation medium and incubated for 18 hours at 23°C in a 1.5 ul sterile conical polypropylene tube. After incubation, the fluid in which the oocytes were cultured is harvested and tested for the presence of IL-1 activity by use of the assays discussed above.

### Preparation of cDNA from mRNA

A library of double-stranded cDNA corresponding to the mRNA, as prepared and assayed above, is constructed by known techniques employing the enzyme reverse transcriptase. One such procedure which may be employed in conjunction with the present invention is detailed by Maniatis et al., supra at 230. Briefly, the polyadenylated mRNA is reverse transcribed by using oligo-dT, that has been hybridized to the polyadenylated tail of the mRNA, as a primer for a first cDNA strand. This results in a "hairpin" loop at the 3' end of the initial cDNA strand that serves as an integral primer for the second DNA strand. Next, the second cDNA strand is synthesized using the enzyme DNA polymerase I and the hairpin loop is cleaved by S1 nuclease to produce double-stranded cDNA molecules. The double-stranded cDNA is fractionated by any convenient means to remove the shorter strands, thereby avoiding the needless cloning of small cDNA fractions.

It is to be understood that in accordance with the present invention, alternative standard procedures may be employed to prepare double-stranded cDNA from mRNA. One such alternative technique is disclosed by Land et al., 9 Nucl. Acids Res. 2251 (1981). In the Land et al. protocol, the hairpin loop is not used as a primer for the second cDNA strand. Rather, the 3' end of the first cDNA strand is tailed with dCMP residues using terminal deoxynucleotidyl transferase ("TdT"). This produces a 3' tail of poly-C residues. Then the synthesis of the second strand is primed by oligo-dG hybridized to the 3' tail. This technique is said to help avoid losing portions of the 5' tail of the second cDNA strand which might occur if the hairpin is cleaved with S1 nuclease, as in the Maniatis et al. protocol.

### Cloning of cDNA

Next, the double-stranded cDNA is inserted within a cloning vector which is used to transform compatible prokaryotic or eukaryotic host cells for replication of the vector. Thereafter, the transformants are identified and plasmid DNA prepared therefrom.

To carry out the present invention, various cloning vectors may be utilized. Although the preference is for a plasmid, the vector may be a bacteriophage or a cosmid. If cloning occurs in mammalian cells, viruses also can be used as vectors.

If a plasmid is employed, it may be obtained from a natural source or artificially synthesized. The particular plasmid chosen should be compatible with the contemplated transformation host, whether a bacteria such as E. coli, yeast, or other unicellular microorganism. The plasmid should have the proper origin of replication for the particular host cell to be employed. Also, the plasmid should have a phenotypic property that will enable the transformed host cells to be readily identified and separated from cells that do not undergo transformation. Such phenotypic characteristics can include genes providing resistance to growth inhibiting substances, such as an antibiotic. Plasmids are commercially available that encode genes resistant to various antibiotics, including tetracycline, streptomycin, sulfa drugs, penicillin and ampicillin.

If E. coli is employed as the host cell, many possible cloning plasmids are commercially available which may be used in conjunction with the present invention. A preferred plasmid for performing the present invention is pBR322. This plasmid has been fully sequenced, as set forth in Sutcliffe, 43 Cold Spring Harbor Symp. Quant. Biol. 77 (1979). A significant advantage of this plasmid is that it has 11 known unique restriction sites, including the Pst I site in the ampicillin resistant gene. This feature is particularly useful for cloning by the homopolymer tailing method.

If a bacteriophage is used instead of a plasmid, such phages should have substantially the same characteristics noted above for selection of plasmids This includes the existence of a phenotypic marker and ligatable termini for attachment of foreign genes.

Preferably, in the present invention, the double-stranded cDNA, having blunt ends, is inserted into a plasmid vector by homopolymeric tailing. As is well known in the art, in this technique, complementary homopolymer tracks are added to the strands of the cDNA and to the plasmid DNA. The vector and double-stranded cDNA are then joined together by hydrogen bonding between complementary homopolymeric tails to form open, circular hybrid molecules capable of transforming host cells, such as E. coli.

In one procedure for homopolymeric tailing, approximately 50 to 150 dA nucleotide residues are added to the 3' ends of linearized plasmid DNA. A similar number of dT nucleotide residues are added to the 3' ends of the double-stranded cDNA and then the cDNA and plasmid joined together.

In an alternative and preferred method, dG tails are added to the 3' ends of the cloning vector that has been cleaved with an appropriate restriction enzyme. For instance, if the pBR322 plasmid is employed, the restriction enzyme Pst I may be used to digest the plasmid at the ampicillin resistant gene. Complementary dC tails are added to the 3' ends of the double-stranded cDNA prior to insertion of the cDNA segment in the plasmid with an appropriate annealing buffer.

It is to be understood that the double-stranded cDNA may be inserted within plasmid cloning vectors by other various standard methods. One such alternative technique involves attaching synthesized nucleotide linkers to the ends of the cDNA strands by using DNA ligase. The linkers are cleaved with a restriction enzyme to generate cohesive termini for insertion within a plasmid cleaved with the same restriction enzyme. Scheller et al., 196 Science 177-180 (1977); Maniatus et al., supra at 219.

The recombinant DNA plasmids, as prepared above, are used to transform host cells. Although the host may be any appropriate prokaryotic or eukaryotic cell, it is preferably a well-defined bacteria, such as E. coli or a yeast strain. Such hosts are readily transformed and capable of rapid growth in culture. Other forms of bacteria, such as salmonella or pneumococcus, may be substituted for E. coli. In place of bacteria, other unicellular microorganisms may be employed, for instance, fungi and algae. Whatever host is chosen, it should not contain a restriction enzyme that would cleave the recombinant plasmid.

If E. coli is employed as a host, preferable strains are MM294 and RR1. Protocols for transformation of the MM294 host by a plasmid vector are well known, as set forth in Maniatis et al., supra at 255; and, Hanahan, 166 J. Mol. Biol. 557 (1983). Protocols for transformation of the RR1 host by a plasmid vector are also well known as set forth in Bolivar et al., 2 Gene 95 (1977) and Peacock et al., 655 Biochem. Biophys. Acta. 243 (1981). Other strains of E. coli which also could serve as suitable hosts include DH1 (ATCC No. 33849) and C600. These strains and the MM294 and RR1 strains are widely commercially available.

In transformation protocols, including those disclosed by Maniatis et al., supra, and Hanahan, supra, only a small portion of the host cells are actually transformed, due to limited plasmid uptake by the cells. The cells that have been transformed can be identified by placing the cell culture on agar plates containing suitable growth medium and a phenotypic identifier, such as an antibiotic. Only those cells that have the proper resistance gene (e.g., to the antibiotic) will survive. If the recombinant pBR322 plasmid is used to transform E. coli strain MM294, transformed cells can be identified by using tetracycline as the phenotypic identifier.

### Preparation of a Synthetic Oligonucleotide Screening Probe

A radiolabeled synthetic oligonucleotide corresponding to part of the N-terminal portion of the amino acid sequence of human IL-1 molecule, as determined above, is used as a probe to screen the cDNA library. The hybridization of the synthetic oligonucleotide probe with plasmid cDNA prepared from the library clones is subsequently identified by autoradiography.

The N-terminal portion of the amino acid composition of IL-1 molecule was determined above as composed of the residues: NH₂-Ala-Pro-Val-Arg-Ser-Leu-Asn-Cys-Thr-Leu-Arg-Asp-Ser-Gly-Gln-Lys-Ser-Leu-Val-Met-Ser-Gly-Pro-Tyr-Glu-Leu-Lys-Ala-Leu-His-Leu-Gln-Gly-Gln-Asp-Met-Glu-Gln-Gln- Val. This sequence information is employed as the basis for the synthetic oligonucleotide probe.

Applicants developed a synthetic oligonucleotide from the above amino acid sequence for use as a probe to screen plasmid DNA thought to contain the IL-1 gene. The probe is composed of the following sequence which corresponds to the antisense sequence coded for by the above amino acid sequence downstream from the first Met residue: 5'-AC TTG TTG TTC CAT GTC TTG GCC TTG CAG GTG CAG GGC TTT CAG TTC GTA GGG GCC GGA CAT-3'. This probe has the advantage of being short enough to be easily synthesized, while being long enough to contain sufficient information to be useful as a probe for the IL-1 gene. Although the described oligonucleotide sequence is a preferred composition of the synthetic probe of the present invention, it is to be understood that probes of other compositions corresponding to other segments of N-terminal amino acid sequence of the IL-1 molecule can be employed without departing from the spirit or scope of the present invention.

The synthetic oligonucleotide probes may be chemically synthesized by well-known techniques, such as by phosphodiester or triester methods. The details of the triester synthesis technique are set forth in Sood et al., 4 Nucl. Acid Res. 2557 (1977); and, Hirose et al., 28 Tet. Lett. 2449 (1978). After synthesis, the oligonucleotide probe is labeled with T4 polynucleotide kinase and ³²P-ATP. A standard protocol from the labeling procedure is set forth in Maniatis et al., supra at 122. Advantageously, the oligonucleotide probes can be synthesized with OH 5' termini thereby avoiding the phosphatase procedure typically required.

### Screening of cDNA Library

In the screening procedure of the present invention, the transformants are pooled into groups each composed of approximately 2,000 transformants. The replicated plasmids are extracted from the transformants using any one of several well-known techniques, such as by alkaline lysis. Plasmid DNA is prepared by cleaving the plasmids at the Pvu II and Hind III restriction sites, both being unique sites on the hybrid plasmid. The resulting DNA segments are fractionated by electrophoresis on agarose gel and then directly analyzed by Southern blotting as described in Southern, 98 J. Mol. Biol. 503 (1975). The DNA that binds to the nitrocellulose filter in the Southern blotting procedure is hybridized with the labeled oligonucleotide probe. The specific DNA fragments that hybridize to the probe are identified by autoradiography.

The particular pool(s) of clones that give a signal following autoradiography are plated out and used in direct bacterial colony hybridization on a nitrocellulose filter with the same above-identified oligonucleotide probes. After completion of the hybridization, the nitrocellulose filter is monitored by autoradiography to identify the largest colony. In the present invention, applicants discovered one such colony. Plasmid DNA designated as IL-1 X-14 is prepared from the particular positive colony identified.

### Characterization of Screened cDNA

The plasmid DNA prepared above is characterized by restriction enzyme mapping. Various strategies for restriction enzyme mapping are discussed by Maniatis et al., supra at 374. One standard technique involves the partial digestion of end-labeled fragments of linear DNA. This technique was developed by Smith and Birnstiel, 3 Nucl. Acids Res. 2387 (1976). A partial restriction enzyme map of the IL-1 X-14 plasmid in the region of the IL-1 gene is shown in FIGURE 3. The distance between restriction sites is given in base pairs ("bp"). The Pst I restriction sites shown in the brackets are those generated by the cloning procedures.

The mapped plasmid cDNA illustrated in FIGURE 3 was sequenced using the chain-termination method. This method of nucleotide sequencing was originated by Sanger et al., 70 Proc. Natl. Acad. Sci. (USA) 5463 (1977). See also U.S. Patent No. 4,322,499. Methods for chain-termination sequence determination are set forth in the Amersham Handbook entitled, M13 Cloning and Sequencing, Blenheim Cresent, London (1983) (hereinafter "Amersham Handbook"); Messing, 2 Recombinant DNA Technical Bulletin, NIH Publication No. 79-99, 2, 43-48 (1979); Norrander et al., 26 Gene 101 (1983); Cerretti et al., 11 Nucl. Acids Res. 2599 (1983); and, Biggin et al., 80 Proc. Natl. Acad. Sci. (USA) 3963 (1983). M13 filamentous phage are employed as vectors to clone the DNA sequences of interest. These phage vectors provide single-stranded DNA templates which are readily sequenced by chain-termination method, which involves priming a single-stranded template molecule with a short primer strand having a free 3' hydroxyl group and then using DNA polymerase to copy the template strand in a chain extension reaction using all four deoxyribonucleotide triphosphates, i.e., dATP, dCTP, dGTP, and dTTP (collectively referred to as "dNTPs"), with one of them being radiolabeled. In the synthesis reaction, a nucleotide specific chain terminator lacking a 3'-hydroxyl terminus, for instance, a 2', 3' dideoxynucleotide triphosphate ("ddNTP"), is used to produce a series of different length chain extensions. The terminator has a normal 5' terminus so that it can be incorporated into a growing DNA chain, but lacks a 3' hydroxyl terminus. Once the terminator has been integrated into the DNA chain, no further deoxynucleotide triphosphates can be added so that growth of the chain stops. Four separate synthesizing reactions are carried out, each having a ddNTP of one of the four nucleotide dNPTs, i.e., dATP, dCPT, dGTP and dTTP. One of the normal dNTPs is radiolabeled so that the synthesized strands after having been sorted by size on a polyacrylamide gel, can be autoradiographed. The chain extensions from the four reactions are placed side by side in separate gel lanes so that the pattern of the fragments from the autoradiography corresponds to the DNA sequence of the cloned DNA.

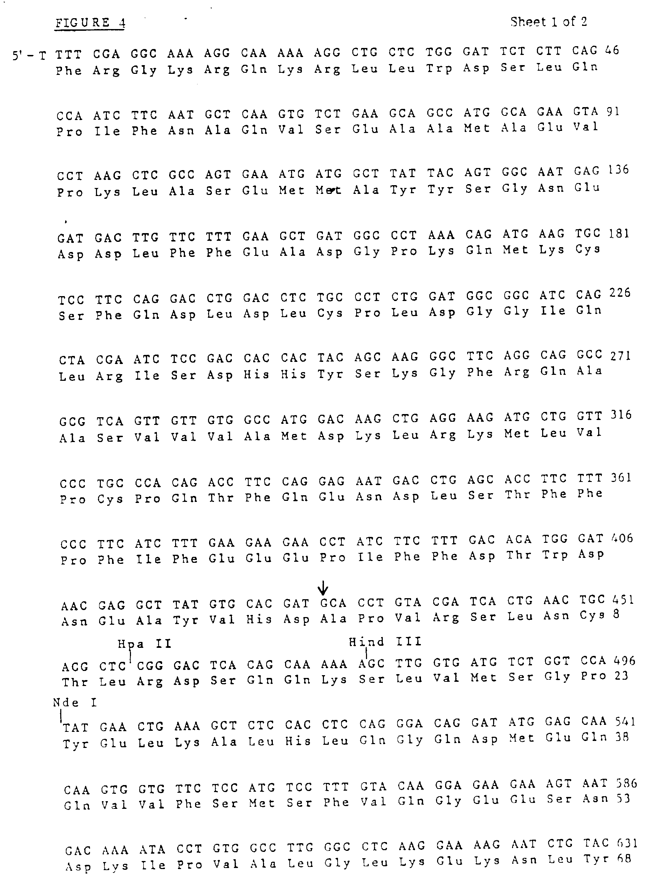

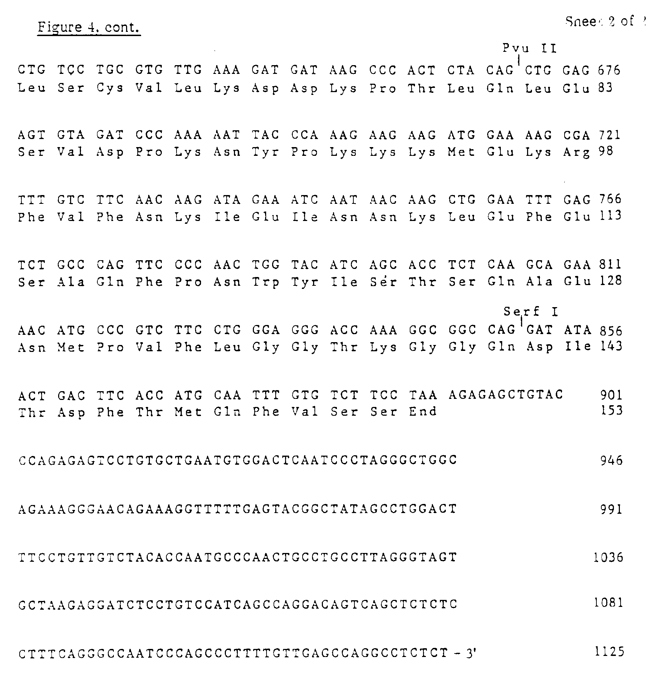

The DNA and corresponding amino acid sequences of the plasmid cDNA in FIGURE 3, as determined by the above techniques, is illustrated in FIGURE 4. The nucleotides are numbered from the beginning of the sequence shown in FIGURE 4. The amino acids are numbered beginning from the mature NH₂-terminus of the IL-1 protein, i.e., the Ala residue, marked with an arrow, and extending to the Ser residue (No. 153) located adjacent the termination codon TAA. The coding region of the IL-1 gene, extending from the Ala codon to the TAG termination codon, is shown as a box portion in FIGURE 3. The restriction enzyme cleaving sites identified in FIGURE 3 are also indicated in FIGURE 4.

In preparation for the sequencing procedures, the plasmid cDNA section shown in FIGURE 3 is digested with various restriction endonucleases and then the resulting DNA fragments cloned into M13 phage vectors to form single stranded DNA templates. A universal primer is used to sequence upstream and downstream from intermediate locations of the sense and antisense strands. Rather than relying on the sequencing results obtained from sequencing the entire length of the fragments with a single chain termination procedure, additional synthetically produced primers are used to initiate the chain termination procedure from other intermediate locations along the lengths of the strands. By this process, both strands of the plasmid cDNA shown in FIGURE 3 are sequenced in overlapping fashion, thereby serving to redundantly confirm the sequences.

It is to be understood that rather than employing the chain-termination technique outlined above, other known methods may be utilized to sequence the IL-1 gene without departing from the spirit or scope of the present invention. For instance, the chemical degradation method of Maxam and Gilbert as set forth in 74 Proc. Nat'l Acad. Sci. (USA) 560 (1977) can be used.

Amino acid sequence studies of IL-1 prepared as above and purified were conducted according to the method of Stern et al., Proc. Natl. Acad. Sci. (USA) 871 (1984). The endopeptidase, cyanogen bromide was used to cleave the IL-1 at the methionine residues and then the resulting fragments analyzed by standard Edman degradation method. By this procedure, applicants have confirmed that the C-terminal of the IL-1 protein is composed of the amino acid squence: Gln-Phe-Val-Ser-Ser. This establishes that the "natural" IL-1 is not processed by removal of amino acids from this end of the molecule after translation from mRNA. This is important because it is clear that much of the coded protein is removed from the 5' end of the open reading frame of the IL-1 gene during the maturation of IL-1 from its precursor.

### Expression of Functional IL-1 From cDNA Clone

To determine whether the cDNA coding region of the IL-1 X-14 clone could encode functional IL-1, the clone is expressed in a prokaryotic/eukaryotic host system. A hybrid cDNA fragment containing the coding region of the IL-1 X-14 clone is inserted into a shuttle expression vector having two sets of replication sequences, a first sequence for amplification of the vector in prokaryotic host cells, and a second sequence for high level expression of the foreign structural protein, i.e., IL-1, in eukaryotic host cells. The transformed eukaryotic host cells are harvested and assayed for expression of mature IL-1 by use of the above detailed thymocyte proliferation assay and IL-2 conversion assay.

Various types of shuttle vectors have been developed. A common type includes an origin of replication and promoter sequences that signal DNA replication in prokaryotic cells, typically E. coli and a comparable origin of replication and promoter sequences that signal DNA replication in eukaryotic cells, most commonly yeast cells. The shuttle vector also includes a phenotypic marker, such as a drug resistant gene, for selection of the transformed prokaryotic cells. The shuttle vector has a comparable phenotypic marker change for selection of transformed eukaryotic cells. Ideally, for high level expression of IL-1, all protein coding sequences are removed from the eukaryotic promoter sequence to avoid expression of an undesired protein. Also, to this end, a natural or synthetic initiator codon sequence, i.e., ATG, is attached to the 5' end of the inserted coding region of the IL-1 gene.

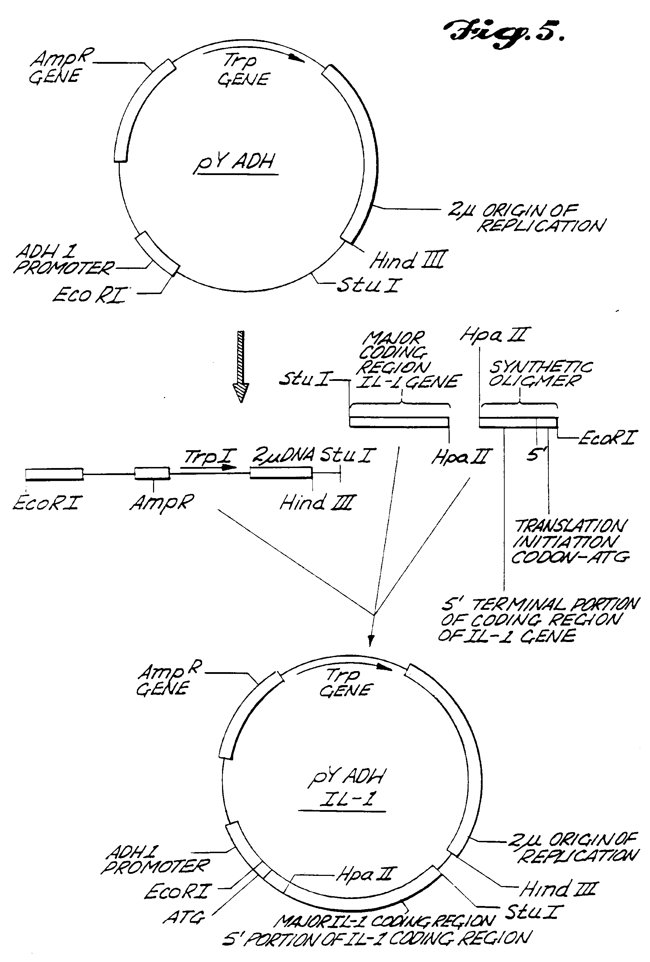

A preferable shuttle vector for carrying out the present invention is designated as pY ADH. As illustrated schematically in FIGURE 5, the pY ADH plasmid includes an origin of replication (from plasmid pBR322) for high copy DNA expression in E. coli, and an ampicillin ("Amp^{R}") resistant gene for selection of transformed E. coli cells. The shuttle vector also includes the 2u circle origin of replication and a yeast Trp I gene for selection of transformed yeast hosts in yeast (trp minus) auxotrophs. The shuttle vector further includes the yeast promoter sequence from the alcohol dehydrogenase gene ("ADH") for propagation of the plasmid in both yeast and E. coli hosts. This promoter sequence is especially advantageous for use in the present invention due to the high level expression of this gene in yeast, and because the complete DNA sequence of this gene is known. All protein coding sequences, including the initiator ATG codon, have been removed from the ADH promoter fragment. The pY ADH shuttle vector includes a number of unique substrate sites for cleavage with restriction enzymes, i.e., Eco RI and Stu I.

As illustrated in FIGURE 5, the pY ADH IL-1 plasmid is prepared as an expression vector for expression of IL-1 gene by insertion of the coding region of the IL-1 gene in plasmid pY ADH. Samples of this shuttle vector are on deposit with the American Type Culture Collection ("ATCC"), 12361 Parklawn Drive, Rockville, Maryland 20852, under Accession No. 39967. This deposit was made on 21st December 1984 and is of plasmid pY ADH IL-1 in E.coli strain RR1. The coding region of the IL-1 gene is removed from the cDNA plasmid, prepared above. Due to the absence of a unique restriction enzyme cleavage site at precisely the 5' end of the coding region of the IL-1 gene, a major portion of the coding region is cleaved from the plasmid cDNA with the restriction enzymes Hpa II and Pst I. The Hpa II site is located slightly downstream from the 5' end of the gene coding region. Thereafter, a synthetic oligonucleotide containing the cleaved 5' end of the gene is chemically synthesized with a Hpa II cohesive 3' terminal for convenient ligation to the "natural" major IL-1 cDNA fragment. Since, as noted above, all protein coding sequences downstream from the ADH promoter sequence were removed, the synthetic oligonucleotide is synthesized with an ATG initiation codon at its 5' end.

The IL-1 cDNA fragment together with the synthetic oligonucleotide are inserted in shuttle vector pY ADH which previously has been digested with appropriate restriction enzymes corresponding to the configurations of the 5' terminal of the synthetic oligonucleotide and the 3' terminal of the major IL-1 cDNA fragment. The resultant recombinant shuttle vector pY ADH IL-1 is used to transform a prokaryotic host, e.g., E. coli, for high copy amplification of the shuttle vector. After this initial transformation process, the recombinant shuttle vector is isolated from the E. coli host and then employed to transform a eukaryotic host, e.g., yeast cells, for high level expression of IL-1. The transformed yeast hosts are harvested and the resulting supernatant is assayed for biological activity utilizing the above described thymocyte proliferation and/or IL-1 conversion assays.

The processes and products of the present invention are further illustrated by the following examples.

### EXAMPLE 1

### IL-1 Production

Leukocyte concentrates of a volume of 350 - 400 ml, obtained from human whole blood (mixture from Portland, Oregon Red Cross), were mixed with and diluted in Ca⁺⁺, Mg⁺⁺ free phosphate buffered saline ("PBS") layered onto Histopaque (Sigma Chemical Company, St. Louis, MO) and then centrifuged at 600 x g for 30 minutes at room temperature. The interface layer, consisting of the leukocytes, was recovered, washed with PBS and centrifuged at 400 x g for 10 minutes at room temperature. The cells were washed two more times in Ca⁺⁺, Mg⁺⁺ free PBS and centrifuged at 200 x g for 10 minutes after each washing.

The resulting mononuclear cells in a concentration of 2 x 10⁶ cells/ml, were cultured in spinner flasks in MEM Medium. The medium was supplemented with 50 U/ml penicillin, 50 ug/ml streptomycin, 2 mM glutamine, 0.2 mM gentamycin, 10 mM HEPES, pH 7.4. The cells were stimulated into IL-1 production by the addition of 0.01 mg/ml of heat inactivated, formalin fixed, Staphylococcus aureus (Igsorb, The Enzyme Center, Inc., Malden, MA). To reduce the production of contaminating proteins, serum was not added to the culture medium. Following incubation for 24 hours at 35°C in a humidified atmosphere of 5% CO₂ in air, the culture was centrifuged at 7000 x g for 30 minutes at room temperature and then the supernatant removed and stored in polyproplylene bottles at -20°C until used.

### EXAMPLE 2

### Ion Exchange Chromatography

IL-1 containing supernatants, as prepared in Example 1, were purified by cation exchange chromatography and anion exchange chromatography. These chromatography procedures were performed at 4°C and the gels used therein were pretreated with 0.1% Triton-X and 10% v/v fetal calf serum to reduce non specific absorption of IL-1 activity to the resins. Prior to the chromatography procedures, the culture supernatant was assayed, as described above. The crude IL-1 solutions were found to have a typical total activity of 1.98 x 10⁷U, a specific activity of 6.37 x 10⁴U/mg of sample and a total protein content of 3.11 x 10⁵ug.

### A. Cation Exchange Chromatography

The ionic strength of the culture supernatant was adjusted by addition of 1 M sodium citrite buffer, pH 4.0, to a final concentration of 10 mM citrite and also was reduced to a pH of 4.0 with concentrated HCl. The supernatant as thus adjusted was applied to 30 x 1.6 cm column of sulfopropyl Sephadex ("SPS") C-25 (Pharmacia Fine Chemicals, Piscataway, NJ) which had been previously equilibrated with the same buffer together with 150 mM NaCl, pH 4.0. The culture supernatant was applied to the column at a rate of 400 ml/hour.

After loading was completed, the column was washed with 10 column volumes of 10 mM 2-N-morpholino ethanesulfonic acid ("MES") buffer, pH 5.0, to remove unbound protein. The bound protein was then eluted from the column with four column volumes of 10 mM Tris-HCl, pH 8.1, applied to the column at a rate of 50 ml/hour. The pH of a column was found to rise after application of approximately three column volumes of the eluant thereby resulting in elution of the IL-1 peak. Column fractions were collected and assayed, as discussed above.

Applicants found that the IL-1 eluted from the cation exchange column exhibited an activity of approximately 1.1 x 10⁷U and specific activity of approximately 2.10 x 10⁵U/mg thereby achieving approximately a three fold increase in IL-1 activity while retaining approxmately 56% of the initial IL-1. Also, approximately 80% of the contaminating proteins were removed by the cationic exchange chromatography procedure.

### B. Anion Exchange Chromatography

The pooled concentrate from the cation exchange column was further purified by anion exchange chromatography on a column of DEAE-Sephacel (Pharmacia Fine Chemicals, Piscataway, NJ). The DEAE-Sephacel column was equilibrated with 10 mM Tris-HCl, pH 8.1. Since the IL-1 was eluted off the SPS column with this same equilibration buffer, the SPS pool was loaded directly onto the DEAE-Sephacel column at a rate of 20 ml/hr, thereby avoiding any loss of activity by dialysis. After loading, the column was washed with five column volumes of the same equilibrating buffer and then elution was carried out with a linear gradient of four column volumes of 0-400 mM NaCl in 10 mM Tris-HCl, pH 8.1.

Applicants found that the IL-1 activity eluted in a sharp peak at 0.08-0.12 M NaCl. SDS-PAGE analysis of the elution fractions revealed some high molecular weight contaminants as well as three major bands of molecular weights of approximately 17,500, 15,000 and 12,000 daltons. Analysis of the column eluate revealed a total activity of 7.75 x 10⁶U, a specific activity of 2.58 x 10⁶U/mg, total protein of 3 x 10³ug and a yield of approximately 39%.

### EXAMPLE 3

### Affinity Chromatography

The active fractions from Example 2 was pooled for further purification by affinity chromatography technique employing a 10 x 1.6 cm column of the dye-ligand "Procion" red dye coupled to an agarose matrix (Bethesda Research Laboratories, Bethesda, MD, Cat. No. 5926 SA) that had been preequilibrated in 10 mM Tris-HCl buffer, pH 8.1. To optimize the binding of the IL-1 to the dye-ligand column, the ionic strength of the DEAE-Sephacel pool was lowered to below 40 mM by dilution of the pool 1:4 in 10 mM Tris-HCl buffer, pH 8.1. The dye-ligand column was initially washed with four column volumes of the same starting buffer to remove unbound protein and then elution is carried out with a linear gradient composed of fifteen column volumes of 0 to 1.0 M NaCl in 10 mM Tris-HCl buffer, pH 8.1. Applicants have found that the IL-1 activity typically was eluted in a sharp peak at 0.50 - 0.55 M NaCl. Column fractions were collected and assayed as described above.

SDS-PAGE analysis of the IL-1 active fractions revealed a single protein band having a molecular weight of approximately 17,500 daltons. The high molecular weight bands, noted above, were eluted in the flow through from column and the lower molecular weight bands of 15,000 and 12,000 daltons were eluted at a higher salt concentration. Assays conducted on the active IL-1 fractions revealed a total activity of the IL-1 of about 6.2 x 10⁶U, a specific activity of about 9.5 x 10⁸U/mg, and a total protein content of approximately 6.5 ug. This equates to an overall purity of IL-1 of greater than 99% and a yield of approximately 31% from the starting supernatants. It is clear from the single protein band which resulted from the SDS-PAGE and silver staining of the fractions collected after dye-ligand affinity chromatography, and also from the specific activities of the fractions analyzed, that essential homogenity of the IL-1 molecule was achieved by the present invention while maintaining a high yield of the IL-1.

The homogeneous IL-1 obtained from the dye-ligand affinity chromatography was also analyzed by two-dimensional polyacrylamide gel electrophoresis together with silver staining, as noted above. This procedure consistently yielded four staining spots at exactly the same molecular weight, i.e., 17,500 daltons and stained the same color in silver stain. The spots were always in the same position and the same relative proportions regardless of which lot of starting supernatant was used. The most intense spot has indicated a pl of 6.3-5.9. The other three spots stain less intensely as the pH gradient becomes more acid. These results indicate that human IL-1 exists in vivo in different charged states, perhaps caused by different states of amidation. The fact that the silver staining yields the exact same color for each spot indicates that the same parent protein most likely is staining in all cases.

### EXAMPLE 4

### Affinity Chromatography-Blue Dye

As an alternative to Example 3, active fractions from Example 2 are pooled and then further purified by essentially the same affinity chromatography technique used in Example 3 with the exception that the dye ligand was composed of Cibacron® Blue 36A dye cross-linked to an agarose matrix (Bethesda Research Laboratories, Bethesda, MD, Cat. No. 5904 SA). Assays conducted on the active fractions revealed a total activity of the IL-1 of about 5.0 x 10⁶ U and a specific activity of about 7.6 x 10⁸ U/mg, which is approximately 80% of the activity present in the red dye derived IL-1. To achieve greater purity, the active fractions from the blue dye ligand affinity chromatograhy can be collected and the process repeated.

### EXAMPLE 5

### Amino Acid Composition Analysis

Purified IL-1 from Example 3 or 4 was boiled in vacuo in 5.7 N HCl (redistilled from concentrated HCl (Kodak, Rochester, NY)) for twenty-four and forty-eight hours to cause hydrolysis of peptide bonds and release of free amino acids. After hydrolysis, samples were dried under vacuum and then resuspended in 0.2 N sodium citrate, pH 2.2. The samples were then injected into a single column LKB Model 4150-Alpha (Cambridge, England) amino acid anaylzer which employs ninhydrin detection. The areas of the output "peaks" corresponding to the quantities of particular amino acids present were integrated with an LKB Model 2220 recording integrator.

The amino acid composition of human IL-1 as determined by the present technique is set forth in Table I below. As indicated by Table I, no glucosamine or galactosamine has been observed from the above amino acid analysis which corresponds with applicants' prior findings that human IL-1 migrates as a single molecular weight species on polyacrylamide gels. One or both of these observations would likely be contrary if human IL-1 includes attached carbohydrate moieties. Thus, it is unlikely that human IL-1 is a glycoprotein.

**TABLE I**

| Amino acid analysis of human IL-1 | |
|---|---|
| Amino Acid | No. of Residues per Molecule |
| Asp/Asn | 14 |
| Thr | 8 |
| Ser | 11 |
| Glu/Gln | 22 |
| Pro | 6 |
| Gly | 11 |
| Ala | 8 |
| Cys | 1* |
| Val | 11 |
| Met | 4 |
| Ile | 6 |
| Leu | 11 |
| Tyr | 4 |
| Phe | 11 |
| His | 3 |
| Lys | 13 |
| Arg | 3 |

| | |
|---|---|
| * Determined from amino acid sequence analysis. | |

### EXAMPLE 6

### Amino Acid Sequence Analysis of N-Terminal Portion of Molecule

Fractions containing homogenious IL-1 from the dye-ligand affinity chromatography procedures of Examples 3 or 4 initially were diluted 10-fold with deionized distilled water, adjusted to pH 4.0 with 1 N HCL and then applied to a 0.5 ml bed volume SPS C-25 chromatography column. Prior to the application of the IL-1, the column was equilibrated with a buffer composed of 0.1 M sodium citrate, 0.05 M NaCl, pH 4.0. After loading of the IL-1, the column was washed with 10 ml of the same equilibration buffer followed with 20 ml of deionized, distilled water. Thereafter, the IL-1 was eluted with 10 mM sodium borate, pH 9.0.

In a first sequencing analysis procedure, 11.1 ug of homogeneous IL-1, as concentrated above, was dried under vacuum and then subjected directly to automated amino terminal Edman degradation using an Applied Biosystems Model 470 protein sequencer. By this process applicants discovered that the N-terminal portion of the IL-1 molecule is composed of the following sequence of amino acid residues: NH₂-Ala-Pro-Val-Arg-Ser-Leu-Asn-Cys-Thr-Leu-Arg-Asp-Ser-Gln-Gln-Lys-Ser-Leu-Val-Met

In a second sequencing analysis procedure the homogeneous Il-1 was fractionated with the enzyme trypsin and then subjected to automated sequencing analysis. Prior to fractionation of the IL-1 molecules with trypsin, the side chains of the lysine residues were blocked with the specific agent citraconic anhydride. To this end, fractions from the SPS C-25 column eluate were concentrated in vacuo to a final volumn of 1 ml. One ul of citraconic anhydride (Pierce) was added to the concentrated IL-1 and then the pH adjusted to 8.3 with 5 N NaOH. The reaction mixture was stirred for 15 minutes and allowed to stand at room temperature for 1 hour. At that time, a second 1 ul quantity of citraconic anhydride was added, the reaction mixture was stirred for an additional 15 minutes and then the mixture was adjusted to final pH of 8.3 with 5 N NaOH. After standing at room temperature for an additional hour, 100 ul of 1 M Tris-HCl, pH 7.4 and 100 ul of 1 M NH₄HCO₃, pH 7.8 were added to the reaction mixture to complete the blocking process.

Next, 2 ug of TPCK-treated trypsin (Worthington) (in 10 ul of 10 mM HCl, pH 2.0) was added to the reaction mixture to cleave the IL-1 molecule at the arginine residues. After addition of the trypsin, the mixture was gently stirred for 5 seconds and then incubated at 37°C for two hours. After completion of the incubation period, an additional 2 ug of TPCK-treated trypsin was added and the mixture incubated at 37°C for an additional 2 hours. The mixture was then cooled to room temperature and adjusted to pH 2.5 with 90% formic acid (Baker). After standing for 4 hours at room temperature, the acidified mixture was diluted by adding 0.5 ml of 6 M guanidine-HCl and then injected onto a 4.6 x 250 mm Vydac 218 TP column, which previously had been equilibrated with 0.1% TFA (v/v) in water, at a now rate of about 1 ml/min. with a Beckman Model 112 pump (Beckman Instruments, Division of Smith Kline Beckman). The loaded column was initially washed with 0.1% TFA (v/v) in water to remove non-bound components and then the IL-1 peptides were eluted from the column with a linear gradient of 0-100% acetonitrile in 0.1% TFA (v/v) at a rate of 0.5%/min. at a flow rate of 1 ml/min. The elution of the peptides was detected by ultraviolet light spectrophotometry at 230 nanometers wavelength.

Individual fractions or pools composed of two or more fractions containing IL-1 peptides were sequenced by automated amino terminal Edman degradation. Prior to the sequencing, the HPLC fractions containing IL-1 were analyzed by gel electrophoresis to determine the number of peptides in each fraction. Thereafter, the peptides were concentrated in vacuo to final volumes of approximately 30 ul, spotted onto a conditioned filter and then dried in a heating chamber at 44°C. The dried filter was placed into an Applied Biosystems Model No. 470A automated protein sequencer. By this procedure, applicants discovered that a major fragment of the human IL-1 molecule near the N-terminal portion thereof is composed of the following sequence of amino acid residues: Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe. The first four residues of the N-terminal end of this sequence coincides with the last four residues of the C-terminal end of the sequence determined above by direct automated protein sequencing, leading to the conclusion that this intermediate sequence is a continuation of the N-terminal sequence set forth above.

### EXAMPLE 7

Mononuclear cells prepared as discussed above in the first paragraph of Example 1 were added to plastic culture flasks in RPMI-1640 medium together with 10% fetal bovine serum (v/v). Following a two-hour incubation at 37°C, nonadherent cells were decantled and the flasks were then replenished with additional serum supplemented RPMI-1640 medium containing 20 ug/ml E. coli LPS at 20 ug/ml. Sixteen hours later, adherent LPS stimulated cells were harvested for RNA.

Total RNA was extracted from the adherent mononuclear cells by the method as described by Chirgwin et al., supra. In this procedure guanidinium thiocyanate was used to denature the cellular protein including the RNase at a rate that exceeds the rate of RNA hydrolysis by RNase. The mRNA was removed from the cellular protein by ultracentrifugation through a dense cushion of cesium chloride.

Thereafter, polyadenylated mRNA was separated from the extracted protein on an oligo (dT)-cellulose chromatography column using the method disclosed by Maniatis et al., supra at 197. Briefly, the column was prepared with application buffer composed of 20 mM Tris-Cl (pH 7.6), 0.5 M NaCl, 1 mM ethylene diamine tetraacetate ("ETDA") and 0.1% SDS. The protein pellet was dissolved in water and application buffer and then loaded onto the column. The nonadsorbed material was eluted by initial washings with application buffer followed by additional washings with application buffer containing 0.1 M NaCl. The retained polyadenylated mRNA was eluted with buffers of reduced ionic strength composed of 10 mM Tris-Cl (pH 7.5), 1 mM EDTA and 0.05% SDS. The eluted polyadenylated mRNA was precipitated at -20°C with 1/10 volume sodium acetate (3M, pH 5.2) and 2.2 volumes of ethanol. After elution of the polyadenylated mRNA from the oligo (dT)-cellulose column, the integrity of the polyadenylated mRNA was confirmed by electrophoresis through agarose gels as detailed in Maniatis et al., supra at 199.

The polyadenylated mRNA was sized by electrophoresis through methylmercury agarose. Gel fractions corresponding to different size classes of mRNA were then translated in vitro, either by use of rabbit reticulocyte lysates or by injection in frog X. laevis oocytes as described above. Fluids liberated by either reticulocyte translations or by mRNA injected oocytes were then tested for the presence of IL-1 activity using the assays set forth above. mRNA gel fractions which, when translated in vitro gave rise to IL-1 activity, were selected as a source of mRNA for cDNA construction.

### EXAMPLE 8

### Construction of cDNA Library

A library of double-stranded cDNA corresponding to the mRNA was prepared from the purified mRNA in Example 7 by employing the standard procedure detailed by Maniatis et al., supra at 229. Oligo-dT was hybridized to the polyadenylated tail of the mRNA to serve as the primer for the reverse transcription of the first cDNA strand. The enzyme avian myeloblastosis virus ("AMV") reverse transcriptase synthesized the first DNA strand by using the mRNA as a template. This procedure resulted in a hairpin loop being formed at the 3' end of the initial cDNA strand that served as an integral primer for the second cDNA strand. After the mRNA strand had been degraded with NaOH, the second cDNA strand was synthesized with DNA polymerase I. The hairpin was then removed with nuclease S1 to produce double-stranded cDNA molecules.

The double-stranded cDNA was fractionated into size classes by Sephacryl S-400 (Pharmacia Fine Chemicals) column chromatography and monitored by analysis using alkaline agarose electrophoresis employing endlabeled fragments of pBR322 DNA as molecular-weight markers. DNA strands having a length of less than 500 bp were culled out to avoid needless cloning of these undersized cDNA fractions.

The double-stranded cDNA fractions, as prepared above, were inserted into the Pst I site of the pBR322 plasmid (Pharmacia Fine Chemicals) by the method disclosed by Maniatis et al., supra, beginning at 239. In this procedure the double-stranded cDNA was tailed with poly (dC) at its 3' ends. The plasmid pBR322 was digested with Pst I endonuclease and then tailed with poly (dG) at its 3' ends. The tailed plasmid DNA and the tailed cDNA were annealed with annealing buffer (0.1M NaCl, 10 mM Tris-Cl (pH 7.8) and 10 mM ETDA) to form novel recombinant plasmids. All restriction enzymes described herein are commercially available from New England Biolabs, Beverly, MA.

The recombinant plasmids were transformed into E. coli strain MM294 by using the procedure of Hanahan, supra in which the E. coli cells were prepared by growth in elevated levels of Mg²⁺. The transformation hosts were plated and then transformants were identified by use of tetracycline as a phenotypic identifier. By use of this technique, applicants obtained approximately 2 x 10⁶ independent transformants.

### EXAMPLE 9

### Preparation of Synthetic Oligonucleotide Screening Probes

A synthetic oligonucleotide was employed as a probe in screening the cDNA library prepared as set forth above in Example 8. The probe was composed of the following composition: 5' AC TTG TTG TTC CAT GTC TTG GCC TTG CAG GTG CAG GGC TTT CAG TTC GTA GGG GCC GGA CAT 3'. The oligonucleotide probe was chemically synthesized by triester method as detailed by Sood et al., supra and Hirose et al., supra.

After chemical synthesis has been completed, the 5' ends of the oligonucleotide probes were labeled with ³²P. To facilitate labeling, the 5' ends of the oligonucleotide were synthesized with OH termini, thereby eliminating the phosphatese treatment which typically must be employed when labeling DNA fragments. The labeling protocol included adding 1 ul of the synthetic oligonucleotides to 16 ul of ³²P - ATP (3000 ci/mM), 1 ul (10 U) of T4 polynucleotide kinase and 2 ul of 10 x kinase buffer I. The 10 x kinase buffer I was composed of 0.5 M Tris-Cl (pH 7.6), 0.1 M MgCl₂, 50 mM dithiothreitol, 1 mM spermidine and 1 mM ETDA. The reaction was carried out at 37°C for 30 minutes, and thereafter the synthesized oligonucleotides were extracted with phenol/chloroform. The labeled probes were separated from unlabeled oligonucleotides by chromatography on or centrifugation through Sephadex G-50 columns (Pharmacia Fine Chemicals).

### EXAMPLE 10

### Screening of cDNA Library

To facilitate initial screening of the cDNA library prepared in Example 9 above, the transformed bacteria cultures were pooled into groups each having approximately 2,000 transformants different clones. Plasmid DNA was removed from samples of the host bacteria by standard alkaline lysis method detailed by Ish-Horowicz and Burke, 9 Nucl. Acids Res. 2989 (1981). The isolated plasmids were separated into two fragments. This was accomplished by initially digesting the plasmids to completion with Pvu II and Hind III. To this end, the plasmids were redissolved in 20 ul of 1 x Hind III buffer (7 mM Tris, (pH 7.4), 7 mM magnesium chloride, 60 mM NaCl) and then 1 ul of Pvu II and 1 ul of Hind III restriction endonucleaes are added. This mixture was incubated at 37°C for two hours.

Next, the plasmid digests were fractionated by electrophoresis through 0.8% agarose gel with markers of appropriate size. The agarose gel was blotted onto nitrocellulose filter using the standard method described by Southern, supra. After the transfer process, the filter was air dried and baked for two hours at approximately 80°C under a vacuum to bind the DNA fragments to the nitrocellulose.

The bound DNA was next hybridized with the labeled oligonucleotide probes. Briefly, the baked nitrocellulose was presoaked in 6 x saline sodium citrate ("SSC") (20 X SSC is composed of 175.3 g of NaCl and 88.2 g of sodium citrate in 800 ml of H₂O, with pH adjusted to 7.0 with 10N NaOH) and then incubated at 50°C for 2-4 hours in prehybridization buffer composed of 6 x SSC, 0.5% NP40 detergent, 0.1% sareosyl, 5 x Denhardt's solution (0.02% Ficoll, 0.02% polyvinyl pyrrolidone, 0.02% BSA) and 100 ug/ml denatured salmon sperm DNA (Sigma Type III, sodium salt). The filter was then incubated overnight at 50°C with the ³²P-labeled oligonucleotide probe (10⁶ cpm/ug) (from Example 3) in hybridizing solution as above. After overnight hybridization, the filter was washed extensively with 6 x SSC at room temperature and then for 5 minutes at 50°C with 6 x SSC. After air drying, the filter was subjected to autoradiography at - 70°C.

From the autoradiography, applicants found several hybridizing bands. The pool of clones from which the plasmid DNA that produced the hybridizing bands was plated out and then used in direct bacterial colony hybridization on nitrocellulose paper with the labeled oligonucleotide probe under the same hybridizing conditions as above. By this process, a single positive colony was identified.

### EXAMPLE 11

### Restriction Enzyme Mapping of Screened cDNA

Plasmid, designated as IL-1 X-14, was prepared from the identified positive colony by the procedures set forth in Example 10. Samples of the IL-1 X-14 plasmid transformed into E. coli strain RR1 are on deposit with the ATCC under Accession No. 39925. Thereafter, the IL-1 X-14 plasmid was analyzed by restriction enzyme mapping using the technique developed by Smith and Birnstiel, supra, involving partial digestion of end-labeled fragments of the linearized DNA. The DNA fragments were labeled at their 5' termini with ³²P-phosphoryl groups using polynucleotide kinase and ³²P - ATP. The labeled DNA strands were then cleaved asymmetrically with a suitable restriction enzyme to provide two fragments, each labeled at only one of its ends. These labeled fragments were isolated by gel electrophoresis. Each of the two fragments was partially digested by appropriate restriction enzymes. Although a large spectrum of digestion fragments may be produced, the labeled fragments form a simple overlapping series each having a common labeled terminus. These fragments were fractionated by gel electrophoresis and then examined by autoradiography. The locations of the fragments on the gel correspond directly to the order of the restriction sites along the plasmid DNA.

By this procedure, applicants have partially mapped the restriction sites, as shown in FIGURE 3, of the IL-1 X-14 plasmid in the region of the IL-1 gene. The numbers shown between the restriction sites of the gene correspond to the approximate distances between the sites, in base pairs.

### EXAMPLE 12

### Sequencing of Screened cDNA

The DNA segment shown in FIGURE 3 was sequenced by the dideoxy chain-termination method essentially as described in the Amersham Handbook, supra, with the variations set forth below. The DNA segment was digested with Hind III and Pst I restriction endonucleases and then the resulting DNA fragments were cloned into strains mp18 and mp19 of the M13 singlestranded filamentous phage vector (Amersham, Arlington Heights, Illinois). The mp18 and mp19 phage vectors, as set forth in Norrander et al. supra, contain the following unique cloning sites: Hind III; Sph I; Pst I; Sal I; Acc I; Hinc II; Xba I; BamHI; Xma I; Sma I; Kpn I; Sst I; and, EcoRI. The composition of the mp18 and mp19 vectors are identical, with the exception that the order of the above-identified restriction sites are reversed in the mp19 vector so that both strands of a DNA segment may be conveniently sequenced with the two vectors. The mp18 and mp19 vectors, with fragments of the cDNA segment of FIGURE 3 inserted therein, were used to transform E. coli JM103 and JM105 of the strain K12 (Bethesda Research Laboratories, Bethesda, Maryland) to produce replicate single-stranded DNA templates containing single-stranded inserts of the sense and antisense strands.

The synthetic universal primer: 5'-CCCAGTCACGACGTT-3' (P-L Biochemicals, Milwaukee, Wisconsin), was annealed to the single-strand DNA templates and used to prime DNA synthesis upstream and downstream from a location between nucleotides 476 and 477 (FIGURE 4) as described above at page 19. Thereafter, the extension fragments were size-separated by gel electrophoresis and autoradiographed from which the nucleotide sequences of the fragments were deduced. Three additional primers were employed to prime synthesis from intermediate locations along the sense strands of the DNA segment in FIGURE 4. A primer having the composition: 5'-CTGGAGAGTGTAGATCC-3', corresponding to nucleotides 671 through 688 (FIGURE 4), was used to prime synthesis of the sense strand in the downstream direction from nucleotide No. 688. The composition of this primer strand was established from the sequencing information previously obtained by use of the universal primer. A second synthetic primer of the composition: 5'-GATATAACTGACTTCAC-3' (corresponding to nucleotides 851 through 868 in FIGURE 4) was used in sequencing the sense strand in the downstream direction from nucleotide No. 868. A third primer having the sequence: 5'-GATTCGTAGCTGGATGC-3' (corresponding to nucleotides No. 235 through No. 218) was employed to sequence the antisense strand in the upstream direction from nucleotide No. 218.

By the above "walk down" method, both strands of the plasmid cDNA in FIGURE 3 were sequenced in an overlapping, redundant manner thereby confirming their nucleotide sequence. It is to be understood that other synthetic primers could have been employed to initiate chain extensions from other locations along the strands without departing from the scope of the present invention. The above primer strands were chemically synthesized by triester method as detailed by Sood et al., supra and Hirose et al., supra. It is to be understood, however, that other well-known techniques, such as by phosphodiester method, may be employed to synthesize the primer strands.

Deoxyadenosine 5' (alpha-[³⁵S] thio) triphosphate (hereinafter "dATP [alpha-³⁵S]") was used as the radioactive label in the dideoxy sequencing reactions. Also, rather than using the gel set forth at page 36 of the Amersham Handbook, a 6% polyacrylamide gel was employed (6% polyacrylmide gel, 0.4 mm thick, containing 7 M, urea 100 mM Tris borate (pH 8.1), and 2 mM EDTA).

As noted above, the nucleotide sequence of the plasmid DNA in FIGURE 3 is illustrated in FIGURE 4. This segment of DNA was found to include the region of the IL-1 gene coding for mature IL-1. The nucleotides are numbered from the beginning of the DNA segment in FIGURE 4. The corresponding amino acids, as determined by the nucleotide sequence and by protein sequence analysis, are set forth above the appropriate codons. The amino acid composition of the IL-1 gene extends from the mature NH₂-terminus of the IL-1 molecule, i.e., the Ala residue, as marked with an arrow in FIGURE 4 (from which the numbering of the amino acid residues begins), to the Ser residue (No. 153) immediately preceding the termination codon TAA. Various restriction enzyme cleaving sites are also indicated in FIGURE 4. The coding region of the IL-1 gene in FIGURE 4 is illustrated a a boxed section in FIGURE 3.

Amino acid sequence studies of IL-1 were conducted according to the method of Stern et al., supra, wherein cyanogen bromide was employed to cleave the IL-1 at the methionine residues. The resulting fragments were separated by size by standard ion-exchange methods. The isolated peptide fragments were then sequenced by automated amino terminal Edman degradation using an Applied Biosystems Model 470 protein sequencer. By this process, applicants have confirmed the results obtained by nucleotide sequencing that the C-terminal of the IL-1 protein is composed of the amino acid sequence: Gln-Phe-Val-Ser-Ser. This establishes that the "natural" IL-1 is not processed by removal of amino acids from this end of the molecule after translation from mRNA. This is significant since from the nucleotide sequence of the IL-1 gene in FIGURE 4, it is clear that a significant amount of RNA sequence is removed from the N-terminus of the IL-1 gene during the maturation of IL-1 from its precursors.

### EXAMPLE 13

### Expression of Mature IL-1

The coding region of the IL-1 gene was removed from the cDNA clone of FIGURE 3 and then inserted into the pY ADH shuttle vector to form the recombinant expression plasmid pY ADH IL-1. The restructuring scheme for preparing the pY ADH IL-1 shuttle expression vector is shown in FIGURE 5. This plasmid was amplified in E. coli host cells and then employed to transform yeast host cells for high level expression of mature IL-1. The functionality of the expressed IL-1 was confirmed by using the thymocyte proliferation and IL-2 conversion assays, detailed above.

A major portion of the coding region of the IL-1 gene from the Hpa II site (base #457 in FIGURE 4) to the 3' flanking region of the gene was removed from the cDNA plasmid segment illustrated in FIGURES 3 and 4 by use of the Hpa II and Pst I restriction enzymes in the standard protocol set forth in Maniatis et al., supra at 104. The IL-1 gene segment was cleaved from the cDNA clone at the Hpa II site, which is located 29 nucleotides downstream from the 5' end of the gene, because no convenient restriction site was found to correspond precisely with the 5' terminal of gene. The 3'-Pst I site of the excised IL-1 gene segment was filled in with T4 DNA polymerase to create a blunt end compatible with the Stu I site of the shuttle vector, discussed below.

A synthetic oligonucleotide was chemically synthesized to add back the 5' terminal portion of the coding region of the IL-1 gene and also to create a translation initiation codon at the 5' end of the coding region. The composition of the oligonucleotide, as shown in Table II below, includes an Eco RI cohesive 5' terminal followed by an ATG initiation codon and then the 5' end of the coding region of the IL-1 gene (to the Hpa II site). Although the oligonucleotide shown in Table II was chemically synthesized by triester technique as detailed by Sood et al., supra and Hirose et al., supra, it is to be understood that the oligonucleotide can be prepared by other methods, such as by phosphodiester method.

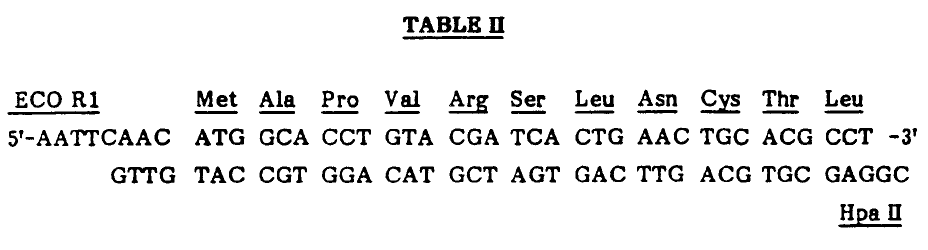

Also, rather than cleaving the coding region of the IL-1 gene at the Hpa II site, the plasmid cDNA in FIGURE 4 could be cleaved at a restriction enzyme site in the 5' flanking region of the gene. Thereafter, the nucleotides of the flanking region can be sequentially removed by standard techniques.

The pY ADH shuttle vector was prepared for ligation to the synthetic oligonucleotide and the excised major portion of the coding region of the IL-1 gene by digestion of the vector to completion with the restriction endonucleases Eco RI and Stu I by standard techniques, as set forth in Maniatis et al., supra at 104. The desired larger fragment from the digestion of the pY ADH plasmid was isolated by electrophoresis on 0.7% agarose gel at 100 volts at 22°C for two hours.

As shown in FIGURE 5, the synthetic DNA oligomer, the excised major portion of the coding region of the IL-1 gene and the desired linearized pY ADH fragment were ligated together in a reaction mixture composed of 100 ug of the pY ADH vector fragment (Eco RI - Stu I), 40 ug of the major IL-1 cDNA fragment (Hpa II, Pst I [blunt]), 5 ug of synthetic oligonucleotide (Eco R I - Hpa II), 1 ul of T4 DNA ligase and sufficient T4 ligase buffer (0.4 M Tris [pH 7.4], 0.1 M MgCl₂, 0.1 M dithiothreitol, 10 mM spermidine, 10 mM ATP and 1 mg/ml BSA) to form a 20 ul reaction volume. The reaction was carried out by incubation at 15°C for 15 hours.

The resulting recombinant plasmid, designated as pY ADH IL-1, was then transformed into E. coli strain RR1 using standard transformation techniques, such as set forth in Bolivar et al., supra and Peacock et al., supra. The host cells were cultured to amplify the pY ADH IL-1 plasmid, and then the plasmids were removed from the host bacteria by standard alkaline method as detailed by Maniatis et al., supra at 368 and by Ish-Horowicz and Burke, supra. The plasmid DNA was purified by centrifugation to equilibrium in cesium chloride-ethidium bromide density gradients, as set forth in Maniatis et al., supra at 93. It is to be understood that other techniques for extracting/concen trating the amplified plasmid DNA from the E. coli may be employed without departing from the scope or spirit of the present invention.

The amplified pY ADH IL-1 plasmid, as prepared above, was then employed to transform the protase deficient yeast strain 20B-12 (alpha, pep 4.3, Trp 1) of S. Cerevisiae by standard techniques. Prior to transformation, the 20B-12 strain was grown in culture in YP-glucose medium (200 ml) to cultures of 2 X 10⁷ cells/ml. The cells were harvested by centrifugation at 1000 X g for 5 minutes at 22°C, and then the resulting pellet was washed with sterile distilled water.

The yeast cells were then concentrated by resuspending in 20 ml of SED (1 M sorbitol, 25 mM ETDA [pH 8.0], and 50 mM dithiothreitol) and incubated for 10 minutes at 30°C. The cell-buffer mixture was then centrifuged for 5 minutes at 300 X g. The pellet was washed once with 200 ml of 1 M sorbitol and the cells resuspended in 20 ml of SCE (1 M sorbitol, 0.1 M sodium citrate [pH 5.8], 0.1 M ETDA). Glusulase, to break down the cell walls, in an amount of 0.2 ml was added to the solution and then the solution incubated at 30°C for 30 minutes with occasional gentle shaking.

The presence of spheroplasts was assayed by diluting 10 ul of yeast cells into a drop 5% sodium dodecyl sulfate (SDS) (wt./vol.) on a microscope slide to observe for "ghosts" at 400 X phase contrast.

The cell mixture was then centrifuged at 300 X g for 3 minutes. The resulting pellet was twice washed with 20 ml of 1 M sorbitol. The pellet was then washed once with STC (1 M sorbitol, 10 mM CaCl, 10 mM Tris HCl [pH 7.5]).

The yeast spheroplasts were then transformed with the previously prepared plasmid vector in a procedure adapted from Beggs, 275 Nature (London) 104 (1978). The pelleted protoplasts are suspended in 1.0 ul of STC and then divided into 100 ml aliquots in 10 ul disposable tubes (Falcon #2059). Then, from 1 to 10 ul of the DNA plasmids were added to each aliquot (0.5 to 5 ug). The mixture was rested at room temperature for 10 minutes and then 1 ml of PEG (20% PEG 4000, 10 mM CaCl₂, 10 mM Tris - HCl [pH 7.4]) was added to each aliquot to promote DNA uptake. After 10 minutes at room temperature, the mixture was centrifuged for 5 minutes at 350 X g. The resulting pellet was resuspended in 150 ul of SOS (10 ml of 2 M sorbitol, 6.7 ul of YEP [0.13 ml of 1 M of CaCl, 27 ul of 1% leucine, and 3.7 ml of H₂O]). This mixture was incubated for 20 minutes at 30°C.

Thereafter, the protoplast/DNA mixture was plated in the presence of yeast minimal medium containing 1.2 M sorbitol and 3% agar at 45°C and without tryptophan. The minimal medium was composed of 0.67 Difco yeast, Nitrogen Base, 0.5% casamino acids, 2% glucose. By maintaining the protoplast/DNA mixture in this medium, only transformants survived, i.e., those that contained the Trp 1 gene.

Prior to biological assay, the transformants were inoculated from the minimal medium into rich medium (1% yeast extract, 2% peptone, 2% glucose) and grown at 30°C for 15-20 hours until the late exponential phase. At the time of harvest, the protease inhibitor phenyl methyl sulfonyl fluoride (PMSF) was added to 1 mM. The culture was then centrifuged at 400 X g to pellet the cells. Thereafter, the cells were washed once in 0.1 vol. cold H₂O. For breakage, the cells were resuspended in 0.01 vol. cold H₂O containing 1 mM PMSF and vortexed with glass beads (1/3 vol.) for 2 minutes. The cell debris and glass beads were pelleted by centrifugation. The resulting supernatant was found to exhibit IL-1 activity. This was ascertained by utilizing the supernate in both of the thymocyte proliferation and IL-1 conversion assays, discussed above.

It will readily be appreciated that alternative sections of the IL-1 gene shown in Figure 4, which sections contain the coding region of the gene, also can be expressed. To this end, the pY ADH IL-1 plasmid can be digested with StuI and Eco R1 restriction endo-nucleases to remove the particular portion of the IL-1 gene identified above, thereby resulting in the original pY ADH plasmid. Thereafter, another section of the IL-1 gene can be ligated to the pY ADH plasmid by standard techniques, as set forth in Maniatus et. al., supra at 104.

## Claims (Claims for the following Contracting State(s): BE, CH, DE, DK, ES, FR, GB, GR, IE, IT, LI, LU, MC, NL, PT, SE)

1. A purified and isolated DNA molecule whose primary translation product, upon direct expression, is a functional mature human IL-1 having IL-1 biological activity in a thymocyte proliferation assay wherein said molecule is derived from a cDNA clone capable of hybridizing to an oligonucleotide probe after overnight hybridization at about 50°C in 6 x saline sodium citrate, wherein said probe comprises the nucleotide sequence:

ACTTGTTGTTCCATGTCTTGGCCTTGCAGGTGCAGGGCTTTCAGTTCGTAGGGG-
CCGGACAT.

2. DNA as claimed in claim 1 and coding for a protein having, for the first 42 amino acids, the amino acid sequence:
NH₂-Ala-Pro-Val-Arg-Ser-Leu-Asn-Cys-Thr-Leu-Arg-Asp-Ser-Gln-Gln-Lys-Ser-Leu-Val-Met-Ser-Gly-Pro-Tpy-Glu-Leu-Lys-Ala-Leu-His-Leu-Gln-Gly-Gln-Asp-Met-Glu-Gln-Gln-Val-Val-Phe.

3. DNA as claimed in claim 1 or claim 2 and having the restriction nuclease map shown in Figure 3.

4. DNA as claimed in any one of claims 1 to 3 and coding for the amino acid sequence extending from residue No. 1 to residue No. 153 inclusive shown in Figure 4.

5. DNA as claimed in any one of claims 1 to 4 and having the nucleic acid sequence extending from nucleic acid base No. 428 to nucleic acid base No. 886 inclusive shown in Figure 4.

6. A recombinant DNA cloning vector comprising DNA as claimed in any of claims 1 to 5.

7. A non-human host transformed by a cloning vector as claimed in claim 6.

8. A process for producing a cloning vector as defined in claim 6, which comprises inserting DNA as defined in any of claims 1 to 5 into a vector under the control of a control sequence and so as to be phenotypically expressible in a host for the vector.

9. A process for producing a transformant capable of expressing an interleukin 1, comprising transforming a non-human host with a cloning vector as defined in claim 6.

10. A process for preparing an interleukin 1, comprising culturing a transformant which has been produced by a process as claimed in claim 9 so as to permit expression of the interleukin 1 gene.

## Claims (Claims for the following Contracting State(s): AT)

1. A process for the preparation of a purified and isolated DNA molecule whose primary translation product, upon direct expression, is a functional mature human IL-1 having IL-1 biological activity in a thymocyte proliferation assay wherein said molecule is derived from a c-DNA capable of hybridizing to an oligonucleotide probe after overnight hybridization at about 50°C in 6 x saline sodium citrate, wherein said probe comprises the nucleotide sequence: 
ACTTGTTGTTCCATGTCTTGGCCTTGCAGGTGCAGGGCTTTCAGTTCGTAGGGG-
CCGGACAT;
 the process comprising coupling successive nucleotides together and/or ligating oligonucleotides.

2. A process as claimed in claim 1, wherein the DNA molecule codes for a protein having, for the first 42 amino acids, the amino acid sequence:
NH₂-Ala-Pro-Val-Arg-Ser-Leu-Asn-Cys-Thr-Leu-Arg-Asp-Ser-Gln-Gln-Lys-Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe.

3. A process as claimed in claim 1 or claim 2 wherein the DNA has the restriction nuclease map shown in Figure 3.

4. A process as claimed in any one of claims 1 to 3, wherein DNA codes for the amino acid sequence extending from residue No. 1 to residue No. 153 inclusive shown in Figure 4.

5. A process as claimed in any one of claims 1 to 4, wherein the DNA has the nucleic acid sequence extending from nucleic acid base No. 428 to nucleic acid base No. 886 inclusive shown in Figure 4.

6. A process as claimed in any one of claims 1 to 4, wherein the DNA has the nucleic acid sequence extending from nucleic acid base No. 80 to nucleic acid base No. 886 inclusive shown in Figure 4.

7. A process for producing a cloning vector, the process comprising inserting DNA preparable by a process as claimed in any one of claims 1 to 6 into a vector under the control of a control sequence and so as to be phenotypically expressible in a host for the vector.

8. A process for producing a transformant capable of expressing an interleukin 1 comprising transforming a non-human host with a cloning vector preparable by a process as claimed in claim 7.

9. A process for preparing an interleukin 1 comprising culturing a transformant which has been produced by a process as claimed in claim 8 so as to permit expression of the interleukin 1 gene.

## Patentansprüche (Patentansprüche für folgende(n) Vertragsstaat(en): BE, CH, DE, DK, ES, FR, GB, GR, IE, IT, LI, LU, MC, NL, PT, SE)

1. Gereinigtes und isoliertes DNA-Molekül, dessen primäres Translationsprodukt nach direkter Expression ein funktionelles reifes Human-IL-1 mit biologischer IL-1-Aktivität in einem Thymozytenproliferations-Assay ist, wobei das Molekül von einem cDNA-Klon abgeleitet ist, welcher befähigt ist, mit einer Oligonukleotid-Sonde nach Hybridisierung über Nacht bei etwa 50°C in 6 x NaCl-Natriumcitrat zu hybridisieren, wobei die Sonde die Nukleotidsequenz: 
ACTTGTTGTTCCATGTCTTGGCCTTGCAGGTGCAGGGCTTTCAGTTCGT-
AGGGGCCGGACAT
 umfaßt.

2. DNA nach Anspruch 1, kodierend für ein Protein, welches für die ersten 42 Aminosäuren die Aminosäuresequenz:
NH₂-Ala-Pro-Val-Arg-Ser-Leu-Asn-Cys-Thr-Leu-Arg-Asp-Ser-Gln-Gln-Lys-Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe aufweist.

3. DNA nach Anspruch 1 oder Anspruch 2, welche die in Figur 3 dargestellte Restriktionskarte aufweist.

4. DNA nach irgendeinem der Ansprüche 1 bis 3, welche für die Aminosäuresequenz kodiert, die von Rest Nr.1 bis Rest Nr. 153 einschließlich in Figur 4 reicht.

5. DNA nach irgendeinem der Ansprüche 1 bis 4, welche die Nukleinsäuresequenz aufweist, die von Nukleinsäurebase Nr. 428 bis Nukleinsäurebase Nr. 886 einschließlich in Figur 4 reicht.

6. Rekombinanter DNA-Klonierungsvektor, umfassend DNA nach irgendeinem der Ansprüche 1 bis 5.

7. Nicht-humaner Wirt, welcher mit einem Klonierungsvektor nach Anspruch 6 transformiert ist.

8. Verfahren zur Herstellung eines Klonierungsvektors nach Anspruch 6, welches umfaßt die Insertion von DNA nach irgendeinem der Ansprüche 1 bis 5 in einen Vektor unter der Kontrolle einer Kontrollsequenz, um so in einem Wirt für den Vektor phänotypisch exprimierbar zu sein.

9. Verfahren zur Herstellung eines Transformanten, der zur Expression eines Interleukin-1 in der Lage ist, umfassend das Transformieren eines nichthumanen Wirts mit einem Klonierungsvektor nach Anspruch 6,

10. Verfahren zur Herstellung eines Interleukin-1, umfassend das Kultivieren eines Transformanten, der mit einem Verfahren nach Anspruch 9 hergestellt wurde, um so die Expression des Interleukin-1-Gens zu gestatten.

## Patentansprüche (Patentansprüche für folgende(n) Vertragsstaat(en): AT)

1. Verfahren zur Herstellung eines gereinigten und isolierten DNA-Moleküls, dessen primäres Translationsprodukt nach direkter Expression ein funktionelles reifes Human-IL-1 mit biologischer IL-1-Aktivität in einem Thymozytenproliferations-Assay ist, wobei das Molekül von einem cDNA-Klon abgeleitet ist, welcher befähigt ist, mit einer Oligonukleotid-Sonde nach Hybridisierung über Nacht bei etwa 50°C in 6 x NaCl-Natriumcitrat zu hybridisieren, wobei die Sonde die Nukleotidsequenz: 
ACTTGTTGTTCCATGTCTTGGCCTTGCAGGTGCAGGGCTTTCAGTTCGT-
AGGGGCCGGACAT
 umfaßt; welches Verfahren die Verknüpfung sukzessiver Nukleotide miteinander und/oder die Ligierung von Oligonukleotiden umfaßt.

2. Verfahren nach Anspruch 1, worin das DNA-Molekül für ein Protein kodiert, das für die ersten 42 Aminosäuren die Aminosäuresequenz:
NH₂-Ala-Pro-Val-Arg-Ser-Leu-Asn-Cys-Thr-Leu-Arg-Asp-Ser-Gln-Gln-Lys-Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe aufweist.

3. Verfahren nach Anspruch 1 oder Anspruch 2, worin die DNA die in Figur 3 dargestellte Restriktionsnukleasekarte aufweist.

4. Verfahren nach irgendeinem der Ansprüche 1 bis 3, worin die DNA für die Aminosäuresequenz kodiert, die von Rest Nr. 1 bis Rest Nr. 153 einschließlich in Figur 4 reicht.

5. Verfahren nach irgendeinem der Ansprüche 1 bis 4, worin die DNA die Nukleinsäuresequenz aufweist, die von Nukleinsäurebase Nr. 428 bis Nukleinsäurebase Nr. 886 einschließlich in Figur 4 reicht.

6. Verfahren nach irgendeinem der Ansprüche 1 bis 4, worin die DNA die Nukleinsäuresequenz aufweist, die von Nukleinsäurebase Nr. 80 bis Nukleinsäurebase Nr. 886 einschließlich in Figur 4 reicht.

7. Verfahren zur Herstellung eines Klonierungsvektors, welches umfaßt die Insertion von DNA, die mit einem Verfahren nach irgendeinem der Ansprüche 1 bis 6 herstellbar ist, in einen Vektor unter der Kontrolle einer Kontrollsequenz, um so in einem Wirt für den Vektor phänotypisch exprimierbar zu sein.

8. Verfahren zur Herstellung eines Transformanten, der zur Expression eines Interleukin-1 in der Lage ist, umfassend das Transformieren eines nichthumanen Wirts mit einem Klonierungsvektor, der mit einem Verfahren nach Anspruch 7 herstellbar ist.

9. Verfahren zur Herstellung eines Interleukin-1, umfassend das Kultivieren eines Transformanten, der mit einem Verfahren nach Anspruch 8 hergestellt wurde, um so die Expression des Interleukin-1-Gens zu gestatten.

## Revendications (Revendications pour l'(les) Etat(s) contractant(s) suivant(s): BE, CH, DE, DK, ES, FR, GB, GR, IE, IT, LI, LU, MC, NL, PT, SE)

1. Molécule d'ADN purifiée et isolée dont le produit de traduction primaire, obtenu par expression directe, est une IL-1 humaine mature fonctionnelle présentant l'activité biologique de l'IL-1 dans un essai par prolifération de thymocytes, la dite molécule étant dérivée d'un clone d'ADNc capable de s'hybrider avec une sonde d'oligonucléotide après hybridation pendant une nuit à environ 50°C dans du citrate de sodium salin 6x, la dite sonde comprenant la séquence de nucléotides:

ACTTGTTGTTCCATGTCTTGGCCTTGCAGGTGCAGGGCTTTCAGTTCGTAGGGGCCG
GACAT.

2. ADN selon la revendication 1, et codant pour une protéine qui présente pour les 42 premiers acides aminés la séquence d'acides aminés:
NH₂-Ala-Pro-Val-Arg-Ser-Leu-Asn-Cys-Thr-Leu-Arg-Asp-Ser-Gln-Gln-Lys-Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe.

3. ADN selon la revendication 1 ou la revendication 2 et présentant la carte, obtenue par nucléase de restriction, présentée dans la figure 3.

4. ADN selon l'une quelconque des revendications 1 à 3 et codant pour la séquence d'acides aminés s'étendant du résidu n° 1 jusqu'au résidu n° 153 inclus, présentée dans la figure 4.

5. ADN selon l'une quelconque des revendications 1 à 4 et présentant la séquence d'acides nucléiques s'étendant de la base d'acide nucléique n° 428 jusqu'à la base d'acide nucléique n° 886 inclus, présentée dans la figure 4.

6. Vecteur de clonage d'ADN recombinant comprenant de l'ADN selon l'une quelconque des revendications 1 à 5.

7. Hôte non humain transformé par un vecteur de clonage selon la revendication 6.

8. Procédé pour la production d'un vecteur de clonage selon la revendication 6, lequel procédé comporte l'insertion d'ADN tel que défini dans l'une quelconque des revendications 1 à 5 dans un vecteur, sous le contrôle d'une séquence de contrôle, de manière à pouvoir être exprimé phénotypiquement dans un hôte du vecteur.

9. Procédé pour la production d'un transformant capable d'exprimer une interleukine 1, lequel procédé comporte la transformation d'un hôte non humain par un vecteur de clonage selon la revendication 6.

10. Procédé pour la préparation d'une interleukine 1, qui comprend la mise en culture d'un transformant qui a été produit par un procédé selon la revendication 9, de manière à permettre l'expression du gène de l'interleukine 1.

## Revendications (Revendications pour l'(les) Etat(s) contractant(s) suivant(s): AT)

1. Procédé de préparation d'une molécule d'ADN purifiée et isolée dont le produit de traduction primaire, obtenu par expression directe, est une IL-1 humaine mature fonctionnelle présentant l'activité biologique de l'IL-1 dans un essai par prolifération de thymocytes, la dite molécule étant dérivée d'un clone d'ADNc capable de s'hybrider avec une sonde d'oligonucléotide après hybridation pendant une nuit à environ 50°C dans du citrate de sodium salin 6x, la dite sonde comprenant la séquence de nucléotides: 
ACTTGTTGTTCCATGTCTTGGCCTTGCAGGTGCAGGGCTTTCAGTTCGTAGGGGCCG
GACAT;
 le procédé comprenant l'étape consistant à coupler ensemble des nucléotides successifs et/ou à lier des oligonucléotides.

2. Procédé selon la revendication 1, dans lequel la molécule d'ADN code pour une protéine qui présente pour les 42 premiers acides aminés la séquence d'acides aminés:
NH₂-Ala-Pro-Val-Arg-Ser-Leu-Asn-Cys-Thr-Leu-Arg-Asp-Ser-Gln-Gln-Lys-Ser-Leu-Val-Met-Ser-Gly-Pro-Tyr-Glu-Leu-Lys-Ala-Leu-His-Leu-Gln-Gly-Gln-Asp-Met-Glu-Gln-Gln-Val-Val-Phe.

3. Procédé selon la revendication 1 ou la revendication 2 dans lequel l'ADN présente la carte, obtenue par nucléase de restriction, présentée dans la figure 3.

4. Procédé selon l'une quelconque des revendications 1 à 3, dans lequel l'ADN code pour la séquence d'acides aminés s'étendant du résidu n° 1 jusqu'au résidu n° 153 inclus, présentée dans la figure 4.

5. Procédé selon l'une quelconque des revendications 1 à 4, dans lequel l'ADN présente la séquence d'acides nucléiques s'étendant de la base d'acide nucléique n° 428 jusqu'à la base d'acide nucléique n° 886 inclus, présentée dans la figure 4.

6. Procédé selon l'une quelconque des revendications 1 à 4, dans lequel l'ADN présente la séquence d'acides nucléiques s'étendant de la base d'acide nucléique n° 80 jusqu'à la base d'acide nucléique n° 886 inclus, présentée dans la figure 4.

7. Procédé de production d'un vecteur de clonage, le procédé comprenant les étapes consistant à insérer de l'ADN pouvant être préparé par un procédé tel que revendiqué dans l'une quelconque des revendications 1 à 6, dans un vecteur sous le contrôle d'une séquence de contrôle, de manière à pouvoir être exprimé phénotypiquement dans un hôte du vecteur.

8. Procédé pour la production d'un transformant capable d'exprimer une interleukine 1, qui comprend la transformation d'un hôte non humain par un vecteur de clonage pouvant être préparé par un procédé tel que revendiqué dans la revendication 7.

9. Procédé pour la préparation d'une interleukine 1, qui comprend la mise en culture d'un transformant qui a été produit par un procédé selon la revendication 8, de manière à permettre l'expression du gène de l'interleukine 1.
